(19) **Europäisches Patentamt / European Patent Office / Office européen des brevets**

(11) **EP 3 286 678 B1**

(12) **FASCICULE DE BREVET EUROPEEN**

(45) Date de publication et mention de la délivrance du brevet:
**29.10.2025 Bulletin 2025/44**

(21) Numéro de dépôt: **16726914.1**

(22) Date de dépôt: **21.04.2016**

(51) Classification Internationale des Brevets (IPC):
**G01N 33/569** (2006.01)  **H01J 49/16** (2006.01)
**G01N 33/68** (2006.01)  **G16B 99/00** (2019.01)
**G06F 18/20** (2023.01)  **G16B 40/20** (2019.01)
**G16B 40/00** (2019.01)

(52) Classification Coopérative des Brevets (CPC):
**G16B 40/20; G01N 33/569; G01N 33/6848; G06F 18/20; G16B 40/00; G16B 99/00**

(86) Numéro de dépôt international:
**PCT/FR2016/050940**

(87) Numéro de publication internationale:
**WO 2016/185108 (24.11.2016 Gazette 2016/47)**

(54) **PROCÉDÉ D'IDENTIFICATION PAR SPECTROMÉTRIE DE MASSE D'UN SOUS-GROUPE DE MICROORGANISME INCONNU PARMI UN ENSEMBLE DE SOUS-GROUPES DE RÉFÉRENCE**

VERFAHREN ZUR IDENTIFIZIERUNG EINER UNBEKANNTEN MIKROORGANISMUSUNTERGRUPPE AUS EINER REIHE VON REFERENZUNTERGRUPPEN MITTELS MASSENSPEKTROMETRIE

METHOD FOR IDENTIFYING BY MASS SPECTROMETRY AN UNKNOWN MICROORGANISM SUBGROUP FROM A SET OF REFERENCE SUBGROUPS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **24.04.2015 FR 1553731**

(43) Date de publication de la demande:
**28.02.2018 Bulletin 2018/09**

(73) Titulaire: **Biomérieux**
**69280 Marcy-L'Etoile (FR)**

(72) Inventeurs:
- **ARSAC, Maud**
  **42400 Saint-Chamond (FR)**
- **COTTE-PATTAT, Pierre-Jean**
  **01150 Lagnieu (FR)**
- **GIRARD, Victoria**
  **69270 Saint Romain au Mont d'Or (FR)**
- **MONNIN, Valérie**
  **38510 Creys-Mepieu (FR)**

(74) Mandataire: **bioMérieux PI Groupement mandataires**
**bioMérieux**
**69280 Marcy l'Etoile (FR)**

(56) Documents cités:
**EP-A1- 2 600 284    EP-A1- 2 648 133**

- **PETER KUHNERT ET AL: "Identification of animalby MALDI-TOF mass spectrometry", JOURNAL OF MICROBIOLOGICAL METHODS, ELSEVIER, AMSTERDAM, NL, vol. 89, no. 1, 1 February 2012 (2012-02-01), pages 1 - 7, XP028472983, ISSN: 0167-7012, [retrieved on 20120210], DOI: 10.1016/J.MIMET.2012.02.001**
- **ANNA RETTINGER ET AL: "Leptospira spp. strain identification by MALDI TOF MS is an equivalent tool to 16S rRNA gene sequencing and multi locus sequence typing (MLST)", BMC MICROBIOLOGY, BIOMED CENTRAL LTD, GB, vol. 12, no. 1, 27 August 2012 (2012-08-27), pages 185, XP021115255, ISSN: 1471-2180, DOI: 10.1186/1471-2180-12-185**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- CAROLE CASSAGNE ET AL: "Mould Routine Identification in the Clinical Laboratory by Matrix-Assisted Laser Desorption Ionization Time-Of-Flight Mass Spectrometry", PLOS ONE, vol. 6, no. 12, 1 January 2011 (2011-01-01), pages e28425 - e28425, XP055019302, ISSN: 1932-6203, DOI: 10.1371/journal.pone.0028425
- ANJA FREIWALD ET AL: "Phylogenetic classification and identification of bacteria by mass spectrometry", NATURE PROTOCOLS, vol. 4, no. 5, 1 May 2009 (2009-05-01), pages 732 - 742, XP055182663, ISSN: 1754-2189, DOI: 10.1038/nprot.2009.37
- DOMINIK ZIEGLER ET AL: "Ribosomal protein biomarkers provide root nodule bacterial identification by MALDI-TOF MS", APPLIED MICROBIOLOGY AND BIOTECHNOLOGY, vol. 99, no. 13, 18 March 2015 (2015-03-18), DE, pages 5547 - 5562, XP055250244, ISSN: 0175-7598, DOI: 10.1007/s00253-015-6515-3
- SALAUN S ET AL: "Whole-cell spectroscopy is a convenient tool to assist molecular identification of cultivatable marine bacteria and to investigate their adaptive metabolism", TALANTA, ELSEVIER, AMSTERDAM, NL, vol. 80, no. 5, 15 March 2010 (2010-03-15), pages 1758 - 1770, XP026892690, ISSN: 0039-9140, [retrieved on 20091111], DOI: 10.1016/J.TALANTA.2009.10.020
- SASCHA SAUER ET AL: "Classification and Identification of Bacteria by Mass Spectrometry and Computational Analysis", PLOS ONE, vol. 3, no. 7, 30 July 2008 (2008-07-30), pages e2843, XP055114067, DOI: 10.1371/journal.pone.0002843
- ZHENGPING WANG ET AL: "Mass Spectrometric Methods for Generation of Protein Mass Database Used for Bacterial Identification", ANALYTICAL CHEMISTRY, vol. 74, no. 13, 1 July 2002 (2002-07-01), pages 3174 - 3182, XP055250246, ISSN: 0003-2700, DOI: 10.1021/ac015725f
- T. VILLMANN ET AL: "Classification of mass-spectrometric data in clinical proteomics using learning vector quantization methods", BRIEFINGS IN BIOINFORMATICS, vol. 9, no. 2, 1 January 2007 (2007-01-01), pages 129 - 143, XP055054943, ISSN: 1467-5463, DOI: 10.1093/bib/bbn009
- ELENA N. ILINA ET AL: "Application of matrix-assisted laser desorption/ionization time-of-flight mass spectrometry for the study of Helicobacter pylori", RAPID COMMUNICATIONS IN MASS SPECTROMETRY, vol. 24, no. 3, 1 February 2010 (2010-02-01), pages 328 - 334, XP055054942, ISSN: 0951-4198, DOI: 10.1002/rcm.4394

**Description**

**DOMAINE DE L'INVENTION**

L'invention a trait au domaine de la classification de microorganismes, notamment de bactéries, au moyen de la spectrométrie. L'invention trouve particulièrement application dans l'identification de microorganismes au moyen de la spectrométrie de masse, par exemple de type MALDI-TOF (acronyme de « Matrix-assisted laser desorption/ionization time of flight »).

**ETAT DE LA TECHNIQUE**

Il est connu d'utiliser la spectrométrie ou la spectroscopie pour identifier des microorganismes, et plus particulièrement des bactéries. Pour ce faire, un échantillon d'un microorganisme inconnu à identifier est préparé puis un spectre de masse de l'échantillon est acquis et pré-traité, notamment pour éliminer le bruit, lisser le signal et soustraire la ligne de base (communément appelé « baseline »). Une étape de détection des pics présents dans les spectres acquis est ensuite réalisée. Les pics du spectre ainsi obtenus sont alors classifiés à l'aide d'outils de classification associés à des données d'une base de connaissance construite à partir de listes de pics de référence chacune associés à un microorganisme ou à un groupe de microorganismes (souche, classe, ordre, famille, genre, espèce, etc...) identifié.

Plus particulièrement, l'identification de microorganismes par classification consiste classiquement :

- en une première étape de construction, à l'aide d'un apprentissage supervisé, d'un modèle de classification associé à une base de connaissance en fonction de spectres de masses dits « d'apprentissage » de microorganismes dont on connaît au préalable les groupes, plus particulièrement les espèces, le modèle de classification et la base de connaissance définissant un ensemble de règles distinguant ces différents groupes ;
- en une seconde étape d'identification d'un microorganisme particulier inconnu en :

    o faisant l'acquisition d'un spectre de masse de celui-ci ; et
    o en appliquant au spectre acquis le modèle de classification en relation avec la base de connaissance associée construits préalablement afin de déterminer au moins un groupe, plus particulièrement une espèce, auquel le microorganisme inconnu appartient.

Les documents « Identification ofanimal Pasteurellaceae by MALDI-TOF mass spectrometry » de Peter Kuhnert et al., Journal of Mircobiological Methods 89, 2012, « Leptospira spp. strain identification by MALDI TOF MS is an equivalent tool to 16S rRNA gene sequencing and multi locus sequence typing (MLST) » de Anna Rettinger et al., BMC Microbiology, 2012, et « Mould Routine Identification in the Clinical Laboratory by Matrix-Assisted Laser Desorption Ionization Time-Of- Flight Mass Spectrometry » de Carole Cassagne et al., PLOS ONE, 2011 décrivent chacun une identification de ce type.

Typiquement, un appareil d'identification par spectrométrie de masse comporte un spectromètre de masse et une unité informatique de traitement d'information intégrée partiellement ou totalement au spectromètre ou raccordée à ce dernier au travers d'un réseau de communication (e.g. un ou plusieurs ordinateur(s) personnel(s), serveur(s), circuit(s) imprimé(s), processeur(s) de signaux numériques (ou « DSP »), et de manière générale tout système à base de microprocesseurs pouvant recevoir des données, les mémoriser, les traiter et produire en sortie les données traitées, par exemple pour une mémorisation dans une mémoire informatique et/ou pour leur affichage sur un écran, le système pouvant lui-même comprendre une ou plusieurs unité(s) à base de microprocesseur(s) en charge de traitements de données spécifiques et communiquant en elles) recevant les spectres mesurés et mettant en œuvre la seconde étape précitée. Un tel appareil d'identification est par exemple le Vitek® MS commercialisé par le demandeur. La première étape est quant à elle mise en œuvre par le constructeur de l'appareil qui construit la base de connaissance, ainsi que le modèle de classification et l'intègre dans la machine avant son exploitation par un client. D'autre part certains appareils permettent à leurs utilisateurs de mettre au point leurs propres bases de connaissance et modèles de classification associés.

Afin de procéder à l'acquisition d'un spectre de masse d'un échantillon par spectrométrie MALDI-TOF, celui-ci est déposé sur un support comprenant différents emplacements de réception, également appelé plaque. L'échantillon est ensuite recouvert d'une matrice qui permet la cristallisation de l'échantillon.

L'utilisation d'appareil d'identification par spectrométrie de masse nécessite un étalonnage régulier afin de garantir l'exactitude et la précision sur la mesure des masses-sur-charges attendues dans le spectre analysé. Deux techniques classiques existent et sont réalisées en routine afin de garantir ces paramètres.

L'étalonnage ou calibration externe est une technique réalisée en routine sur la plupart des appareils de spectrométrie de masse. Pour cette technique, le dépôt d'un mélange étalon (ou calibrant externe) est réalisé en un emplacement distinct de celui de l'échantillon sur la plaque, support de l'échantillon dans l'appareil. L'étalonnage externe consiste à ajuster l'axe masse sur charge (m/z) des spectres de masse du mélange étalon, dont le contenu est connu, de manière à ce que les pics observés coïncident avec leur position théorique, une liste de pics de référence correspondant à des masses-sur-charges caractéristiques ayant au préalable été définie pour cet étalon. Lors de l'étalonnage externe, la présence des pics de référence correspondant à ces masses-sur-charges caractéristiques est recherchée dans la liste de pics du spectre du mélange étalon, avec une tolérance donnée sur la position attendue. Le spectre du mélange étalon est ensuite réaligné en fonction de la position observée de chacune des masses-sur-charges de référence trouvée. Par la suite, la transformation appliquée afin de réaligner le spectre du mélange étalon est appliquée au spectre de l'échantillon à analyser afin de réaligner sa position sur l'axe m/z.

Cette méthode présente l'avantage de pouvoir travailler sur de très faibles quantités d'échantillons sans risque de suppression du signal. Cependant l'étalonnage externe n'est pas assez précis pour la classification de microorganismes, notamment à des niveaux taxonomiques inférieurs au niveau espèce.

L'étalonnage ou calibration interne est utilisé pour obtenir un maximum de précision de mesure. Cette technique peut être complémentaire à l'étalonnage externe afin d'apporter plus de précision sur la position des masses-sur-charges du spectre. Cet étalonnage est qualifié d'interne car un mélange étalon (ou calibrant interne) est incorporé à l'échantillon à analyser avant l'acquisition. Dans le cadre de la spectrométrie MALDI-TOF, la matrice (Acide $\alpha$-cyano-4-hydroxycinnamique ($\alpha$-HCCA), etc...) est déposée sur l'ensemble échantillon et étalon afin de les co-cristalliser. Ainsi, lors de l'analyse du spectre de masses acquis, l'assignation des masses-sur-charges connues des composés du mélange étalon permet de calculer des constantes de calibration. Ces constantes sont ensuite utilisées pour le calcul des masses-sur-charges des composés inconnus. Cependant, le principal inconvénient de cette méthode est le risque de suppression du signal des ions analytes présents dans l'échantillon par une trop forte concentration du mélange étalon. Dans le cadre d'une méthode de préparation d'échantillon biologique par digestion trypsique les positions des masses-sur-charges correspondant à la trypsine peuvent également être utilisée comme calibrant interne.

Il est connu que l'identification de certaines espèces ou sous-espèces de microorganismes par spectrométrie MALDI-TOF nécessite une précision élevée sur les spectres acquis afin de différencier des groupes d'espèces proches. Plus particulièrement, la distinction d'espèces proches, l'identification de microorganismes au niveau sous-espèce ou au niveau souche (souches de sérotypes différents, souches de pathotypes différents, souches de génotypes différents, etc...) sont notoirement complexes. Ces sous-groupes présentent en effet des spectres très proches ne rendant pas leur distinction possible avec les bases de connaissances et algorithmes de classification mis au point pour l'identification au niveau groupe, par exemple au niveau taxonomique supérieur. Cette limite est notamment due à la résolution atteinte par les appareils de spectrométrie de masse mais également à la variabilité des acquisitions sur un même appareil ainsi qu'entre appareils différents. Par exemple, un décalage de la position des pics des spectres pour plusieurs acquisitions d'un même échantillon, peut être observé. Ce décalage peut être visible par exemple pour des acquisitions d'un échantillon déposé sur un unique emplacement ou sur plusieurs emplacements du support d'échantillon. Cette variabilité entraine une incertitude sur la mesure de masse-sur-charge, non gênante pour l'identification au niveau groupe mais qui empêche d'exploiter une discrimination à des niveaux inférieurs au groupe tel que des sous-groupes, typiquement à des niveaux inférieurs à l'espèce du microorganisme.

## EXPOSE DE L'INVENTION

L'invention a pour objectif de réduire cette variabilité en améliorant la précision de la position des pics des spectres de masse acquis.

L'invention a également pour objectif de proposer un procédé ne modifiant pas les méthodes de préparation d'échantillon existantes et pouvant être directement utilisé avec les protocoles existants, notamment sans utilisation d'étalon externe ou interne supplémentaire.

Un autre objectif de l'invention est de proposer un procédé permettant l'identification de microorganisme au niveau sous-groupe à la suite d'une identification au niveau groupe.

L'invention a ainsi pour objet un procédé d'identification du groupe d'un microorganisme inconnu suivie de l'identification du sous-groupe de ce même microorganisme par spectrométrie de masse.

Dans ce but, l'invention concerne un procédé d'identification par spectrométrie de masse d'un sous-groupe de microorganisme inconnu parmi un ensemble de sous-groupes de référence, comportant :

- Une première étape de construction d'une base de connaissance et d'un modèle de classification par groupe associé à partir d'un ensemble de spectres d'apprentissage de microorganismes identifiées comme appartenant au dit groupe
- Une seconde étape de construction d'une base de connaissance et d'un modèle de classification par sous-groupe associé à partir de l'acquisition d'au moins un ensemble de spectres d'apprentissage de microorganismes identifiées comme appartenant aux dits sous-groupes du groupe comprenant :

o La construction d'un modèle d'ajustement permettant la correction des décalages de masse-sur-charge des spectres acquis à partir de masses-sur-charges de référence communes aux différents sous-groupes

◦ L'ajustement des masses-sur-charges de l'ensemble des listes de pics des spectres d'apprentissage.

◦ La construction d'un modèle de classification par sous-groupe et de la base de connaissance associé à partir des spectres d'apprentissage ajustés

- Une troisième étape de classification à un sous-groupe d'un microorganisme inconnu comportant :

◦ L'acquisition d'au moins un spectre du microorganisme inconnu

◦ La classification dans un groupe dudit spectre selon ledit modèle de classification par groupe et ladite base de connaissance par groupe

◦ L'ajustement des masses-sur-charges de l'ensemble de la liste de pics dudit spectre selon le modèle d'ajustement permettant la correction des décalages de masse-sur-charge du spectre du microorganisme inconnu

◦ La classification dans un sous-groupe dudit groupe par ledit modèle de classification par sous-groupe et la base de connaissance par sous-groupe

- Mémoriser le résultat de la classification et/ou afficher le résultat de la classification sur un écran d'affichage

L'invention permet ainsi l'identification du groupe d'un microorganisme inconnu suivie directement de l'identification du sous-groupe (sous espèce, type de souche...) de ce même microorganisme par spectrométrie de masse, le tout sans procéder à une seconde acquisition du spectre de masse de l'échantillon contenant le microorganisme inconnu ni d'ajout d'étalon interne.

L'invention a ainsi le même effet sur la précision des masses-sur-charges que l'usage d'un étalon interne, et permet de proposer un mode opératoire en routine pour l'utilisateur de l'appareil de spectrométrie de masse identique à une identification au niveau groupe simple. De plus, l'invention se révèle particulièrement économe en temps nécessaire au développement de la base de connaissance au niveau sous-groupe et à la classification en routine de microorganismes inconnus et ce sans couts supplémentaires d'étalon externe ou interne. La majorité des étapes du procédé selon l'invention sont également automatisables afin de limiter le nombre d'interventions nécessaire à la construction du modèle de classification et de la base de connaissance associée, ainsi qu'à l'analyse en routine de microorganismes inconnus.

Par groupe et sous-groupe, on entend une représentation hiérarchique sous forme d'arbre des types de microorganismes de référence utilisés dans la construction des bases de connaissance, par exemple en termes d'évolution et/ou de phénotype et/ou de génotype. Le niveau de sous-groupe correspondant toujours à un sous-ensemble du groupe. Dans le cas de bactéries, le groupe peut ainsi être une espèce au sens des techniques classiques d'analyse, un sous-groupe pourra alors être une sous-espèce du groupe ou encore un phénotype particulier du groupe. Cependant, un groupe peut également être constitué de plusieurs espèces qui ne sont pas distinguées par des techniques classiques d'analyse, chaque sous-groupe correspondant pourra ainsi correspondre à une ou plusieurs de ces espèces.

Avantageusement, une étape d'optimisation de la liste des masses-sur-charges de référence basée sur la qualité de l'ajustement obtenu suite à au moins une des étapes d'ajustement peut être réalisée.

L'identification et la sélection de masses-sur-charges de référence communes aux différents sous-groupes peut être obtenues à partir de masses-sur-charges connues à priori ou déduites selon des critères statistiques de fréquence de la présence des pics dans chacun des sous-groupes du groupe.

Pour cela, le procédé selon l'invention peut comprendre une étape consistant à

- Discrétiser l'espace des masses-sur-charges de chacun des spectres de chaque sous-groupe
- Détecter la présence ou l'absence de pics autour des masses-sur-charges définies par l'étape de discrétisation selon un facteur de tolérance
- Filtrer lesdites masses-sur-charges en fonction de la fréquence de présence de pics pour chacun des sous-groupes
- Approximer la position des masses-sur-charges retenues

L'étape de discrétisation peut avantageusement être réalisée sur un intervalle de masses-sur-charges restreint par rapport à l'intervalle de masses-sur-charges obtenu suite à l'acquisition du spectre. L'étape d'approximation peut avantageusement consister à rechercher une position représentative de la répartition des positions des pics présents autour de chacune des masses-sur-charges retenues.

L'identification des masses-sur-charges de référence du procédé peut ainsi reposer sur une analyse statistique de la fréquence de présence des pics des spectres acquis pour la construction d'une base de connaissance des sous-groupes, à la fois pour la mise au point du modèle de classification et son utilisation en routine.

Avantageusement, le procédé comprend lors de l'étape de construction d'une base de connaissance et d'un modèle de classification par sous-groupe associé :

- La construction d'un second modèle d'ajustement permettant la correction des décalages de masse-sur-charge des spectres acquis à partir de masses-sur-charges de référence communes aux différents sous-groupes
- Une seconde étape d'ajustement des masses-sur-charges de l'ensemble des listes de pics des spectres d'apprentissage à partir du second modèle d'ajustement

Avantageusement, le procédé comprend une étape de contrôle de l'ajustement suite à au moins une des étapes d'ajustement des masses-sur-charges lors de l'étape de construction d'une base de connaissance et d'un modèle de classification par sous-groupe associé.
Les paramètres du ou des modèle(s) d'ajustement peuvent avantageusement être obtenus par une méthode d'estimation dite robuste.
Avantageusement, les masses-sur-charges de référence communes aux différents sous-groupes connues sont sélectionnées par une étape consistant à

- Détecter la présence ou l'absence de pics autour des masses-sur-charges de référence selon un facteur de tolérance

- Filtrer lesdites masses-sur-charges en fonction de la fréquence de présence de pics pour chacun des sous-groupes et/ou approximer la position des masses-sur-charges de référence retenues

Avantageusement, l'étape de construction d'une base de connaissance et d'un modèle de classification par sous-groupe associé comprenant une étape de discrétisation des masses-sur-charges des spectres acquis.
Avantageusement, l'étape de construction d'une base de connaissance et d'un modèle de classification par sous-groupe associé comprenant une étape de traitement des intensités des spectres acquis.
Avantageusement, l'étape de construction d'une base de connaissance et d'un modèle de classification par sous-groupe associé comprenant une étape de contrôle de la qualité des spectres acquis
Selon un mode de réalisation, la spectrométrie de masse est une spectrométrie MALDI-TOF.
L'invention a également pour objet un dispositif d'identification d'un sous-groupe de microorganisme par spectrométrie de masse, comprenant :

- un spectromètre de masse apte à produire des spectres de masse de microorganismes à identifier ;
- un système informatique apte à identifier un sous-groupe de microorganisme associé aux spectres de masse produits par le spectromètre en mettant en œuvre un procédé conforme à l'une quelconque des objets précédentes.

L'invention a également pour objet un dispositif d'identification d' un sous-groupe de microorganisme par spectrométrie de masse, comprenant :

- un spectromètre de masse apte à acquérir au moins un spectre de masse d'un microorganismes à identifier ;
- un système informatique apte à identifier le microorganismes associé au au moins un spectre de masse acquis par le spectromètre, ledit système comprenant :

  - une mémoire informatique mémorisant :

    o une base de connaissance et un modèle de classification par groupes de microorganismes, associé à partir d'un ensemble de spectres d'apprentissage de microorganismes identifiés comme appartenant auxdits groupes ;
    o une base de connaissance et un modèle de classification par sous-groupes de microorganismes, associé à partir de l'acquisition d'au moins un ensemble de spectres d'apprentissage de microorganismes identifiés comme appartenant auxdits sous-groupes du groupe;
    o un modèle d'ajustement pour la corrections de décalages de masse-sur-charge des spectres acquis par le spectromètre de masse à partir de références communes aux différents sous-groupes de la base de connaissance et du modèle de classification par sous-groupes ;
    o des instructions informatiques pour la production d'une liste de pics à partir du spectre de masse acquis du microorganisme inconnu ;
    o des instructions informatiques pour la classification du microorganisme dans un groupe en fonction de la liste de pics produite selon ledit modèle de classification par groupes et ladite base de connaissance par groupes ;
    o des instructions informatiques pour l'ajustement de la liste de pics selon le modèle d'ajustement ;

o des instructions informatiques pour la classification du microorganisme dans un sous-groupe en fonction de la liste de pics ajustée selon ledit modèle de classification par sous-groupes et ladite base de connaissance par sous-groupe ;

- unité informatique à base de microprocesseur pour la mise en œuvre des instructions informatiques mémorisées dans la mémoire informatique de manière à classifier le microorganisme dans un groupe et un sous-groupe ;
- une mémoire informatique pour mémoriser le résultat de la classification et/ou un écran d'affichage pour afficher le résultat de la classification.

Le système informatique est intégré partiellement ou totalement au spectromètre ou est raccordée à ce dernier au travers d'un réseau de communication, sans fil ou non. Le système comprend par exemple un ou plusieurs ordinateur(s) personnel(s), serveur(s), circuit(s) imprimé(s), processeur(s) de signaux numériques (ou « DSP »), et de manière générale est un système à base de microprocesseurs pouvant recevoir des données, les mémoriser, les traiter et produire en sortie les données traitées, par exemple pour une mémorisation dans une mémoire informatique et/ou pour leur affichage sur un écran, le système pouvant lui-même comprendre une ou plusieurs unité(s) informatique(s) à base de microprocesseur(s) en charge de traitements de données spécifiques et communiquant en elles. Par exemple, une première unité informatique est intégrée dans le spectromètre et est en charge du prétraitement des signaux mesurés (e.g. transformation d'un signal de temps de vol en un signal de masse-sur-charge, tout ou partie du traitement permettant l'obtention des spectres de masse et/ou tout ou partie du traitement permettant l'obtention d'une listes de pics issues des spectres de masse), et une seconde unité informatique distante, ayant par exemple des ressources informatiques plus importantes, est raccordée à la première unité informatique pour la mise en œuvre du reste du traitement menant à l'identification du microorganisme. Il peut par exemple s'agir d'une seconde unité informatique offrant un service de type cloud computing. La mémoire informatique est par exemple une mémoire de masse (e.g. disque dur).

Le dispositif d'identification d'un microorganisme selon l'invention mémorise en outre les données et instructions nécessaires à la mise en œuvre de la troisième étape de classification décrite précédemment.

Par exemple, les données (bases de connaissances, modèle de classification, modèle d'ajustement, etc.) et les instructions sont incorporés à un dispositif d'identification de l'état de la technique qui dispose déjà des ressources informatiques pour mettre en œuvre l'invention. Notamment, l'invention est mise en œuvre par un système d'identification comprenant un Vitek® MS commercialisé par le demandeur

## BREVE DESCRIPTION DES FIGURES

L'invention sera mieux comprise à la lecture de la description qui va suivre, donnée uniquement à titre d'exemple, en relation avec les dessins annexés, dans lesquels :

- la figure 1 est un organigramme du procédé selon l'invention ;
- la figure 2 est un organigramme de l'étape 100 du procédé selon l'invention ;
- la figure 3a est un organigramme de l'étape 200 du procédé selon l'invention ;
- la figure 3b est un organigramme de l'étape 240 du procédé selon l'invention ;
- la figure 3c est un organigramme de l'étape 300 du procédé selon l'invention ;
- la figure 3d est un organigramme de l'étape 400 du procédé selon l'invention ;
- la figure 4 est un tracé pour chaque sous-groupe A à E, d'un groupe considéré, de la fréquence de chaque pic obtenus sur les spectres correspondant au dit sous-groupe dans l'intervalle 5330 Th-5410 Th
- les figures 5a à 5i sont un tracé d'un exemple de calcul itératif en trois itérations de trois masses-sur-charges approximées
- la figure 6 est un tracé pour deux masses-sur-charges Alpha et Béta de la fréquence de présence d'un pic pour chaque sous-groupe A à F, la médiane des résidus pour chaque sous-groupe, l'intervalle interquartile des résidus pour chaque sous-groupe
- les figures 7a et 7b sont un tracé du résultat d'un premier ajustement et second ajustement selon l'invention
- les figures 8a et 8b sont un tracé du résultat d'un premier ajustement et second ajustement selon l'invention
- les figures 9a et 9b sont un tracé du résultat d'un premier ajustement et second ajustement selon l'invention
- les figures 10a et 10b sont un tracé du résultat sur la précision d'un ajustement selon l'invention
- les figures 11a et 11b sont un tracé du résultat sur la précision d'un ajustement selon l'invention
- la figure 12 est un tracé du résultat d'identification au niveau sous-groupe de microorganisme

## DESCRIPTION DETAILLEE DE L'INVENTION

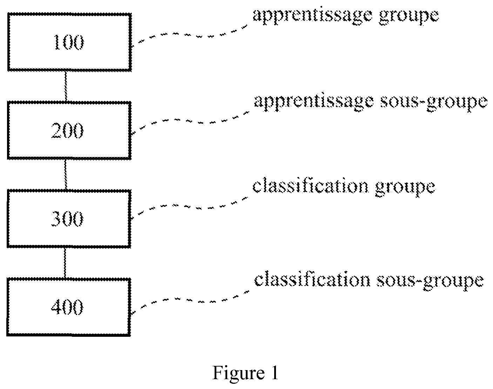

Il va à présent être décrit en relation avec l'organigramme de la figure 1, un procédé selon l'invention.

Le procédé comprend une première étape **100** de construction d'une base de connaissance et d'un modèle de classification par groupe à partir d'un ensemble de spectres d'apprentissage de microorganismes identifiés comme appartenant au dit groupe. De façon générale, cette étape peut être réalisée de multiples manières visant à obtenir pour un ou plusieurs groupe(s) donné(s), une base de connaissance et un modèle de classification permettant de déterminer si un spectre de masse de microorganisme inconnu appartient audit groupe à partir de la liste de pics du spectre acquis. Hormis l'étape **110** décrite ci-après et mise en œuvre par un spectromètre, l'étape **100** est mise en œuvre informatiquement, e.g. au moyen d'un ou plusieurs ordinateur(s) personnel(s), serveur(s), circuit(s) imprimé(s), processeur(s) de signaux numériques (ou « DSP »), et de manière générale tout système à base de microprocesseurs pouvant recevoir des données, les mémoriser, les traiter et produire en sortie les données traitées, par exemple pour une mémorisation dans une mémoire informatique et/ou pour leur affichage sur un écran, le système pouvant lui-même comprendre une ou plusieurs unité(s) à base de microprocesseur(s) en charge de traitements de données spécifiques et communiquant en elles.

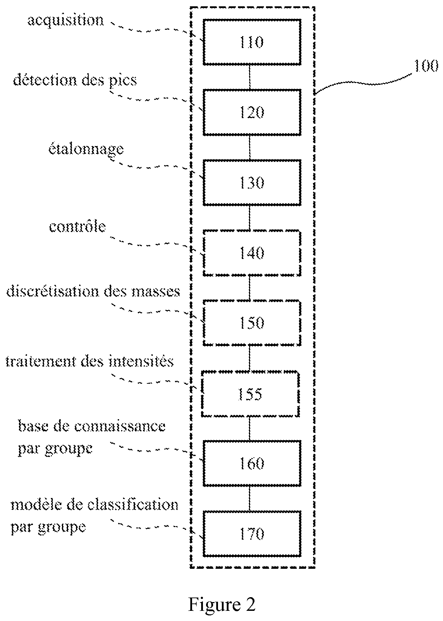

Un exemple de réalisation de cette première étape **100** est détaillé sur la figure 2. L'étape **100** peut ainsi débuter par une étape **110** d'acquisition d'un ensemble de spectres de masse d'apprentissage d'un ou de plusieurs microorganismes identifiés comme appartenant à un groupe, et d'un spectre de masse d'étalonnage externe, au moyen d'une spectrométrie de masse de type MALDI-TOF (acronyme de « *Matrix-assisted laser desorption/ionization time of flight* »). La spectrométrie de masse MALDI-TOF est bien connue en soi et ne sera donc pas décrite plus en détail par la suite. On pourra, par exemple, se référer au document de Jackson O. Lay, « Maldi-tof spectrometry of bacteria », Mass Spectrometry Reviews, 2001, 20, 172-194. Les spectres acquis sont ensuite prétraités, afin notamment de les débruiter, les lisser ou encore d'ôter leur ligne de base si nécessaire, d'une manière connue en soi.

L'acquisition d'un spectre de masse peut consister en la réalisation de plusieurs tirs du laser sur l'échantillon considéré, et ce à une ou différentes positions de l'échantillon sur le support. Le spectre obtenu consiste alors en un spectre « synthétique » obtenu par la sommation, la moyenne, la médiane ou tout autre méthode visant à pondérer la contribution des intensités de chaque spectre de chacun des tirs pour la formation du spectre « synthétique ». Cette accumulation de tirs, bien connue en soit, permet notamment d'augmenter le rapport signal / bruit en limitant l'influence de phénomènes non récurrents dus à l'échantillon, l'appareil, aux conditions de réalisation de l'acquisition, etc...

Une étape de détection des pics présents dans les spectres acquis est alors réalisée en **120,** par exemple au moyen d'un algorithme de détection de pics basé sur la détection de maxima locaux. Une liste des pics pour chaque spectre acquis, comportant la localisation, également appelée la valeur de masse-sur-charge, et l'intensité des pics du spectre, est ainsi produite.

De manière avantageuse, les pics sont détectés dans la gamme de Thomson (Th) $[m_{min} ; m_{max}]$ prédéterminée, de préférence la gamme $[m_{min} ; m_{max}] = [3000;17000]$ Thomson. En effet, il a été observé que les informations suffisantes à l'identification des microorganismes sont regroupées dans cette gamme de rapport masse sur charge, et qu'il n'est donc pas besoin de tenir compte d'une gamme plus large.

Le procédé se poursuit, en **130,** par une étape d'étalonnage externe à partir du spectre de masse d'étalonnage acquis. L'étalonnage (ou calibration externe) consiste à ajuster l'axe m/z des spectres de masse d'un échantillon de référence, dont le contenu est connu, de manière à ce que les pics observés coïncident avec leur position théorique. Une souche d'*Escherichia Coli* sert par exemple d'étalon externe pour détecter les déviations et corriger les décalages de masses-sur-charges. Une liste de pics de référence correspondant à des masses-sur-charges caractéristiques a au préalable été définie pour ce calibrant. Lors de cette étape d'étalonnage, la présence des pics de référence correspondant à ces masses-sur-charges caractéristiques est recherchée dans la liste de pics du spectre, avec une tolérance donnée sur la position attendue. Le spectre est ensuite réaligné en fonction de la position observée. La transformation utilisée pour réaligner les pics de calibrant acquis sur les pics de référence sera ensuite utilisée pour réaligner les pics du spectre de l'échantillon.

Selon un exemple de mise en œuvre de cette étape **130,** pour chaque groupe d'acquisition (par exemple 4x4 emplacements sur un support d'acquisition pour un appareil VITEK® MS commercialisé par la demanderesse) une souche *Escherichia Coli* d'étalonnage (ATCC 8739) est déposée sur l'emplacement réservé à l'étalonnage du dit groupe d'acquisition. Une fois le spectre de la souche d'étalonnage acquis, la présence de 11 pics de référence correspondant à des masses-sur-charges caractéristiques de *Escherichia Coli* est recherchée, avec une tolérance de 0.07 % autour de la position attendue des pics. Si au moins 8 pics sur les 11 se trouvent dans l'intervalle de position attendu, les pics du spectre de la souche d'étalonnage vont être réalignés en fonction de leur position de référence. La transformation utilisée pour réaligner les pics de calibrant acquis sur les pics de référence, par exemple une transformation polynomiale d'ordre un ou deux, sera ensuite utilisée pour réaligner les pics des spectres de tous les autres emplacements du groupe d'acquisition.

Optionnellement et par précaution, l'opération d'acquisition peut être stoppée si un nombre minimal de pics de référence détectés n'est pas atteint. Par exemple si moins de 8 masses-sur-charges caractéristiques sont détectées. Il est également possible d'étendre la tolérance autour des positions des pics de références attendues à 0.15 %. Dans ce cas, si au moins 5 masses-sur-charges caractéristiques sont détectées avec la nouvelle tolérance élargie, il est préférable de réaligner une première fois les pics du spectre de calibrant et de rechercher dans un deuxième temps un nombre de pics de référence plus important avec la tolérance initiale de 0.07 %. Si une quantité plus importante de pics est alors retrouvée, les pics du spectre sont réalignés une seconde fois selon la transformation trouvée.

L'acquisition, le prétraitement et la détection des pics des autres échantillons composant le groupe d'acquisition peut également être réalisée après l'étape d'étalonnage en appliquant la transformation trouvée sur les listes de pics correspondant aux spectres des échantillons. Alternativement, l'étape **130** peut consister ou être complémentée par une étape d'ajustement interne à partir d'un calibrant mélangé à l'échantillon lors de l'étape d'acquisition **110.**

A la suite de l'étape d'étalonnage **130,** le procédé selon l'invention peut comprendre une étape de contrôle de la qualité des spectres acquis **140** et/ou une étape de discrétisation des masses-sur-charges **150** et/ou une étape de traitement de l'intensité des spectres **155.** L'ordre de réalisation de ces étapes **140, 150, 155** pouvant varier.

Optionnellement, le procédé se poursuit donc, en **140,** par une étape de contrôle de la qualité des spectres acquis. Par exemple, il peut être vérifié que le nombre de pics identifiés est suffisant : un nombre trop bas de pics ne permettant pas l'exploitation du spectre acquis pour la classification du microorganisme considéré tandis qu'un nombre trop élevé peut être révélateur de bruit. De façon complémentaire, un test basé sur l'intensité des pics détectés peut également être réalisé lors de cet étape de contrôle de la qualité des spectres.

A la suite de l'étape **130,** optionnellement de l'étape **140,** une étape de discrétisation des masses-sur-charges, ou « binning », des masses-sur-charges **150** peut être réalisée. Pour ce faire la gamme de Thomson [$m_{min}$ ; $m_{max}$] est subdivisée en intervalles de largueur ou « bins », par exemple constants ou constants sur une échelle logarithmique. Pour chaque intervalle comprenant plusieurs pics, un seul pic peut être conservé, avantageusement le pic présentant la plus forte intensité. Cette méthode est donc utilisée pour aligner les spectres et réduire les effets des légères erreurs de position des masses-sur-charges, l'alignement obtenu étant directement lié à la taille des intervalles de discrétisation. Une liste réduite est ainsi produite à partir de chacune des listes de pics des spectres mesurés. Chaque composante de la liste correspond à un intervalle de la discrétisation et a pour valeur l'intensité du pic conservé pour cet intervalle, la valeur « 0 » signifiant qu'aucun pic n'a été détecté dans cet intervalle.

A la suite de l'étape **130,** optionnellement de l'étape **140,** optionnellement de l'étape **150,** une étape **155** de traitement de l'intensité des spectres peut également être réalisée. L'intensité est une grandeur très variable d'un spectre à l'autre et/ou d'un spectromètre à un autre. Du fait de cette variabilité, il est difficile de prendre en compte les valeurs brutes d'intensité dans les outils de classification. Cette étape peut donc être réalisée sur les spectres bruts, avant discrétisation des masses-sur-charges ou après l'étape **150.** Celle-ci peut notamment consister en un étape de seuillage des intensités, les intensités inférieures au seuil étant considérées comme nulles et les intensités supérieures au seuil étant conservées. En variante, les listes d'intensités obtenues par ce seuillage ou suite à une étape de discrétisation peuvent être « binarisées » en posant la valeur d'une composante de la liste à « 1 » lorsqu'un pic est supérieur au seuil ou présent dans l'intervalle de discrétisation correspondant, et à « 0 » lorsqu'un pic est inférieur au seuil ou lorsqu'aucun pic n'est présent dans cet intervalle de discrétisation. Alternativement, les listes d'intensités obtenues sont transformées selon une échelle logarithmique, en posant la valeur de la composante à « 0 » lorsqu'aucun pic n'est présent dans l'intervalle ou lorsqu'un pic est inférieur au seuil. Enfin, une normalisation de chacune des listes d'intensités (ie, brutes, seuillées, « binarisées » ou transformées selon une échelle logarithmique) peut être réalisées.

Avantageusement, les listes d'intensités sont transformées selon une échelle logarithmique puis normalisées. Ceci a pour effet de rendre plus robuste l'apprentissage des algorithmes de classification réalisé ultérieurement.

A partir de ces listes de pics correspondant chacune à un spectre d'apprentissage de microorganisme identifié comme appartenant à un groupe, le procédé se poursuit par la création dans l'étape **160** d'une base de connaissance par groupe et dans l'étape **170** d'un modèle de classification par groupe. La base de connaissance comprenant le paramétrage du modèle de classification et les informations sur les groupes de chaque microorganisme utilisé pour l'apprentissage et permettant de classifier un microorganisme inconnu parmi les groupes des microorganismes d'apprentissage.

Un modèle de classification par groupe est établi dans l'étape **170** à partir d'algorithmes de classification supervisés connus tel que la méthode des plus proches voisins, la régression logistique, l'analyse discriminante, les arbres de classification, les méthodes de régression de type « LASSO » ou « elastic net », des algorithmes de type SVM (acronyme de l'expression anglo-saxonne « *support vector machine* »).

Selon la figure 1, le procédé se poursuit dans l'étape **200** par la construction d'une base de connaissance et d'un modèle de classification par sous-groupe à partir d'un ensemble de spectres d'apprentissage de microorganismes identifiées comme appartenant au groupe précédent et à des sous-groupes de ce groupe. Hormis l'étape **210** décrite ci-après et mise en œuvre par un spectromètre, l'étape **200** est mise en œuvre informatiquement, e.g. au moyen d'un ou plusieurs ordinateur(s) personnel(s), serveur(s), circuit(s) imprimé(s), processeur(s) de signaux numériques (ou « DSP »), et de manière générale tout système à base de microprocesseurs pouvant recevoir des données, les mémoriser, les traiter et produire en sortie les données traitées, par exemple pour une mémorisation dans une mémoire informatique et/ou pour leur affichage sur un écran, le système pouvant lui-même comprendre une ou plusieurs unité(s) à base de microprocesseur(s) en charge de traitements de données spécifiques et communiquant en elles.

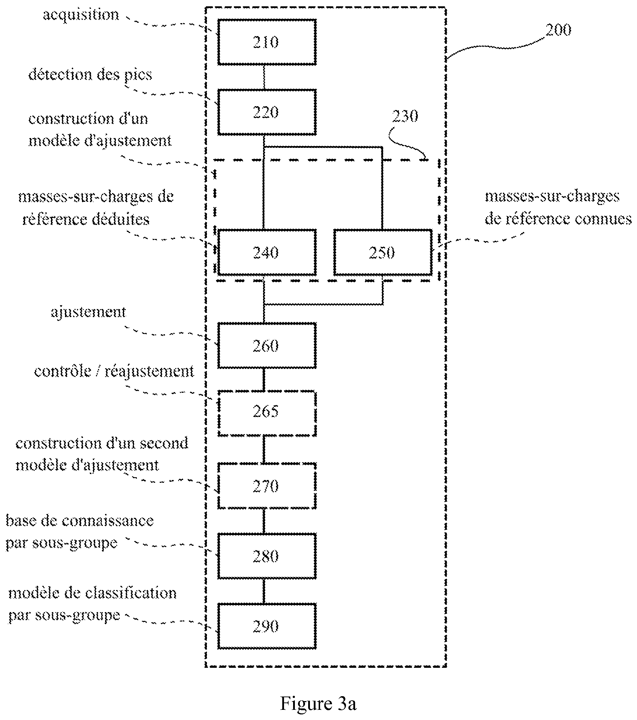

L'étape **200** est détaillée sur la figure **3a.** Cette étape **200** comprend l'acquisition **210** d'au moins un spectre d'un microorganisme dont le groupe et le sous-groupe sont connus et ce pour chacun desdits sous-groupes. Cette étape d'acquisition est réalisée de façon similaire à l'étape **110**. Le spectre acquis est ainsi prétraité, afin notamment de le débruiter, le lisser ou encore d'ôter sa ligne de base si nécessaire. Le procédé se poursuit selon l'étape **220** par l'identification des pics des spectres de façon similaire à l'étape **120,** l'étalonnage externe ou interne de chacun des spectres de façon similaire à l'étape **130,** optionnellement le contrôle de leur qualité de façon similaire à l'étape **140.**

De façon préférentielle, l'étape **210** peut être directement réalisée simultanément à l'étape **110** du procédé afin de limiter le nombre d'étapes manuelles nécessaires aux étapes d'acquisition. Les étapes **110** et **210** consistent alors en une étape unique d'acquisition d'un spectre d'un microorganisme dont le groupe et le sous-groupe sont connus. De la même manière, l'étape **220** est alors réalisée simultanément aux étapes **120** et **130** et éventuellement l'étape **140.**

A la suite de l'étape **220** les spectres des microorganismes dont le groupe et les sous-groupes sont connus sont alors représentés sous la forme d'un ensemble de listes de pics, chaque liste de pics correspondant à un microorganisme dont le groupe et le sous-groupe sont connus.

A partir de ces listes de pics, le procédé se poursuit par une étape **230** de construction d'un modèle d'ajustement permettant la correction des décalages de masse-sur-charge des spectres acquis. Cette étape **230** de construction comporte premièrement une étape d'identification et de sélection de masses-sur-charges de référence communes aux différents sous-groupes. En effet, une masse-sur-charge qui ne serait pas communes aux différents sous-groupe du groupe serait une masse-sur-charge discriminante, un modèle d'ajustement qui se baserait sur cette masse-sur-charge serait donc biaisé. Idéalement, ces masses-sur-charges sont communes aux différents sous-groupes et ne présentent pas de pics à proximité immédiate dans le spectre afin d'obtenir une liste de masses-sur-charges caractérisant particulièrement le groupe.

Selon une première alternative **240,** ces masses-sur-charges de références communes aux différents sous-groupes sont déduites à partir de critères statistiques.

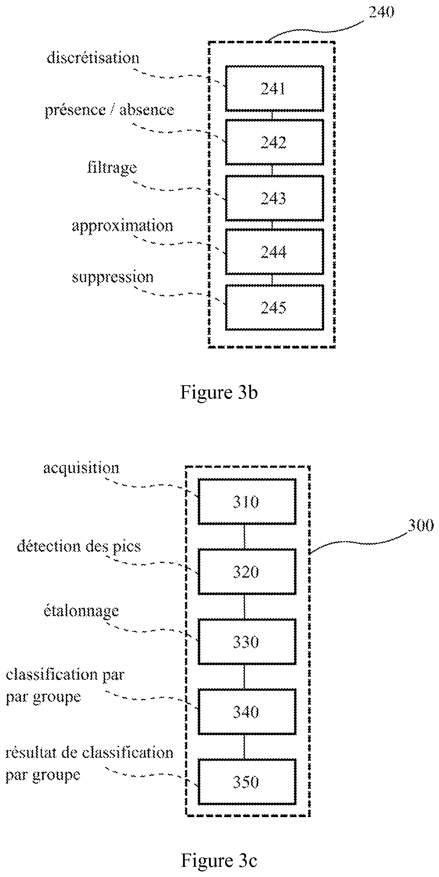

Comme illustré sur la figure **3b,** ces masses-sur-charges de références peuvent notamment être obtenues par :

- Une première étape **241** de discrétisation de l'étendue des masses-sur-charges d'intérêt. Cette étape peut être réalisée sur un intervalle de masses-sur-charges des listes de pics restreint par rapport à l'intervalle de masses-sur-charges obtenu suite à l'acquisition, connu pour contenir l'essentiel des masses-sur-charges caractéristiques des microorganismes, par exemple sur l'étendue de masses-sur-charges 3000 à 17000Th. A partir de cet intervalle, celui-ci est discrétisé :

  ○ soit par intervalle de masse-sur-charge régulier (par ex 1 Th)
  ○ soit par intervalle de masse-sur-charge croissant.

On obtient ainsi un ensemble

$$\{m(i)\} \, ; \, i = 1, \dots, I$$

correspondant à l'ensemble des masses-sur-charges obtenues après discrétisation, chaque valeur *m(i)* étant séparé de la valeur *m*(*i* + 1) par une intervalle de masse-sur-charge appelé pas de discrétisation.

Il est défini un facteur de tolérance $t_1$ définissant un intervalle autour de chacune des masses-sur-charges *m(i).* Pour la bonne réalisation du procédé il est à noter que la discrétisation choisie doit garantir a minima le recouvrement des intervalles définis par le facteur de tolérance $t_1$ d'une masse-sur-charge par rapport à la suivante, idéalement un recouvrement à la moitié de la largeur de l'intervalle. Ainsi, un pas de discrétisation fin est préférable à un pas de discrétisation trop large afin de ne pas écarter une masse-sur-charge qui serait caractéristique des sous-groupes et donc utile pour l'ajustement. Ce pas de discrétisation fin permet donc de limiter la perte d'information.

Une manière de garantir le recouvrement des intervalles d'une masse-sur-charge par rapport à la suivante est de définir la discrétisation itérativement par la formule

$$m(i + 1) = \, m(i) + t_1 * m(i)$$

avec $t_1$ étant le facteur de tolérance, et l'initialisation de *m*(1) à la borne minimale de l'étendue des masses-sur-charges d'intérêt. Le pas de discrétisation est ainsi égal à $t_1$ * *m*(*i*). Par exemple, pour l'étendue des masses-sur-charges d'intérêt de 3000 à 17000 Th avec une tolérance $t_1$=0.0008, le pas de discrétisation à 3000 Th est de 2.4 Th tandis que le pas de discrétisation à 17000 Th est de 13.6 Th.

Une autre manière, plus simple, de garantir le recouvrement des intervalles d'une masse-sur-charge par rapport à la suivante est de définir la discrétisation sur la borne minimale de l'étendue des masses-sur-charges d'intérêt par la formule

$$m(i + 1) \; = \; m(i) + t_1 * m(1)$$

Par exemple, pour l'étendue de masse-sur-charge d'intérêt 3000 à 17000Th avec une tolérance $t_1$=0.0008, le pas de discrétisation applicable à l'ensemble de l'étendue de masse-sur-charge est 3000*0.0008=2.4 Th.

S'en suit une seconde étape **242** de détection de la présence ou absence d'un ou de pics dans l'intervalle selon $t_1$ autour de chaque masse-sur-charge $m(i)$ définie par l'étape de discrétisation. Pour chaque spectre, la tolérance $t_1$ permet de tenir compte de l'incertitude sur la position de la masse-sur-charge recherché dans chacun des spectres acquis.

Ainsi, soit

$$X = \{x(s)\} \, ; \, s = 1, \dots, S$$

la liste des masses-sur-charges du spectre considéré et soit $t_1$ le facteur de tolérance appliqué sur les masses-sur-charges. L'opération consiste à rechercher la présence d'un pic parmi $X = \{x(s)\} \, ; \, s = 1, \dots, S$ dans l'intervalle défini par la tolérance autours de la masse-sur-charge $m(i)$ considérée, à savoir l'intervalle $[m(i) - m(i) * t_1 \, ; \, m(i) + m(i) * t_1]$.

Afin d'optimiser le temps de calcul, la présence d'un pic dans l'intervalle considéré peut être noté 1, l'absence d'un pic ou la présence de plusieurs pics est noté 0 afin d'obtenir une matrice de présence sous la forme du **tableau 1** suivant, T étant le nombre de spectres d'apprentissage acquis:

Tableau 1

|  | Sous-groupe | m(1) | m(2) | ... | m(I-1) | m(I) |
|---|---|---|---|---|---|---|
| Spectre(1) | A | 0 | 0 |  | 1 | 1 |
| Spectre(2) | A | 0 | 0 |  | 1 | 1 |
| ... |  |  |  |  |  |  |
| Spectre(T-1) | B | 0 | 1 |  | 1 | 1 |
| Spectre(T) | B | 1 | 1 |  | 1 | 1 |

A partir de cette matrice, une troisième étape **243** consiste à filtrer les masses-sur-charges en fonction de la fréquence de présence de pics par sous-groupes.

La fréquence de présence d'un pic dans l'intervalle défini par la tolérance autours de chaque masse-sur-charge $m(i)$ définie durant l'étape de discrétisation est calculée par sous-groupe et ramenée à un pourcentage.

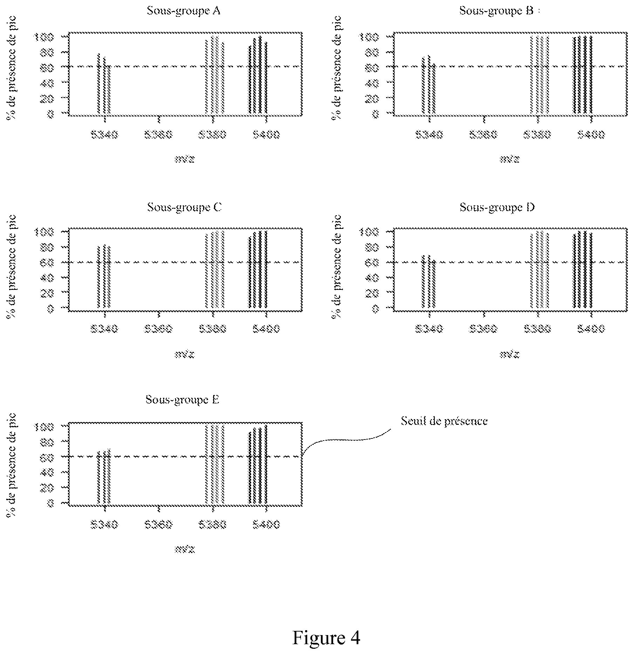

Cette étape est illustré sur la figure **4**. La figure **4** représente pour chaque sous-groupe A à E, du groupe considéré, la fréquence de chaque pic obtenus sur les spectres correspondant au dit sous-groupe dans l'intervalle 5330 Th-5410 Th.

Par la suite, les masses-sur-charges $m(i)$ présentant un pourcentage de présence supérieur à un seuil, par exemple de 60%, représenté par un trait horizontal en pointillé sur la figure 4, pour chacun des sous-groupes à discriminer, sont retenues.

On obtient ainsi un ensemble :

$$\{m(j)\} \, ; \, j = 1, \dots, J; \; J \le I \text{ de masses-sur-charges parmi } \{m(i)\} \, ; \, i = 1, \dots, I$$

retenues après l'étape de filtrage sur la fréquence. Par exemple, selon le tableau 2 ci-dessous, seules les masses-sur-charges m(I-1) et m(I) sont retenues après filtrage.

Tableau 2

| Fréquence (%) par sous-groupe | m(1) | m(2) | ... | m(I-1) | m(I) |
|---|---|---|---|---|---|
| A | 0 | 0 |  | 100 | 100 |
| B | 50 | 100 |  | 100 | 100 |

A partir de cette liste de masses-sur-charges filtrées selon un seuil de fréquence, l'étape suivante **244** consiste à approximer la position desdites masses-sur-charges retenues.

Les masses-sur-charges retenues ont une précision grossière dépendante de la discrétisation menée à l'étape **241**. Une étape d'approximation de la position de ces masses-sur-charges est ainsi réalisée afin d'obtenir une position représentative de la répartition des positions des pics présents autour de la masse-sur-charge $m(j)$. Ce calcul de la position représentative peut par exemple comprendre une étape d'estimation d'une fonction gaussienne représentative de la répartition des pics ainsi que la recherche de la position de l'extremum de cette fonction. Une autre méthode peut consister en la réalisation de plusieurs étapes de calcul itératif de la valeur médiane des positions des pics présents autour de la masse-sur-charge $m(j)$. Pour cette méthode utilisant la médiane, soit $M(j)$ la valeur théorique de la position de la masse-sur-charge .Soit $M(j, 0) = m(j)$, $M(j, n + 1)$ est obtenue par l'algorithme suivant :

Pour chaque spectre, une étape du procédé consiste à rechercher un pic parmi $X = \{x(s)\}$ ; $s$ = 1, ...., $S$ présente dans l'intervalle autour de la masse-sur-charge $M(j, n)$, à savoir l'intervalle $[M(j, n) - M(j, n) * t_2 ; M(j, n) + M(j, n) * t_2 ]$ avec $t_2$ un facteur de tolérance autour de la position de la masse-sur-charge $M(j, n)$, la valeur du facteur de tolérance $t_1$ étant supérieure ou égale à $t_2$.

On obtient ensuite la valeur de $M(j, n + 1)$ en calculant la médiane des valeurs des pics retenus sur l'ensemble des spectres dans l'intervalle autour de $M(j, n)$.

Le critère d'arrêt de cette étape d'optimisation peut être par exemple un nombre prédéfini d'itérations et/ou être basé sur un contrôle de l'incrément.

Par exemple, dans le cas où un nombre prédéfini d'itération est défini :

Soit N le nombre prédéfini d'itérations, $M(j)$ est approximée par $\hat{M}\hat{M}(j) = M(j,N)$.

Dans le cas où le procédé comprend une étape de contrôle de l'incrément, soit $\varepsilon$ une tolérance fixée pour le calcul approché de $M(j)$ . Les itérations s'achèvent dès que :

$$|M(j,n+1) - M(j,n)| < \varepsilon$$

$M(j)$ est alors approximé par $\hat{M}(j) = M(j,n + 1)$

Afin d'assurer la convergence de cette méthode par contrôle de l'incrément et pour économiser le temps de calcul nécessaire à cette étape, un nombre maximal d'itérations N peut également être prédéfini.

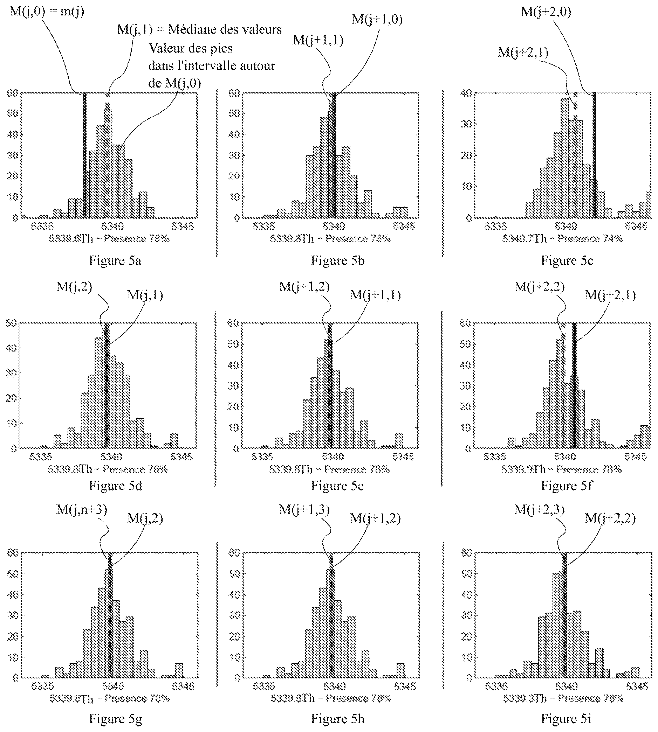

Le critère d'arrêt basé sur un nombre prédéfini d'itérations N=3 est ainsi privilégié pour la mise en œuvre de l'invention. Un exemple de calcul itératif en trois itérations est illustré pour trois masses-sur-charges sur les figures **5a** à **5i.** Sur la figure **5a,** la médiane $M(j, 1)$ calculée à partir des valeurs des pics autour de $M(j, 0)$ est égale à 5339,6 Th et représentée selon un trait vertical pointillé. Dans une seconde itération, illustrée sur la figure **5d,** la médiane $M(j, 2)$ est ainsi calculée à partir des valeurs des pics autour de $M(j, 1)$, une nouvelle valeur égale à 5339,8 Th est alors obtenue. Sur la figure **5d,** $M(j, 1)$ est représentée par un trait vertical plein, $M(j, 2)$ est représentée par un trait vertical pointillé. Dans une troisième itération, illustrée sur la figure **5g,** la médiane $M(j, 3)$ est ainsi calculée à partir des valeurs des pics autour de $M(j, 2)$, une valeur égale à 5339,8 Th est alors encore obtenue, démontrant la convergence de la méthode. Sur la figure **5g** $M(j, 2)$ est représentée par un trait vertical plein, $M(j, 3)$ est représentée par un trait vertical pointillé. Le calcul est stoppé sur cette troisième itération est la valeur approximée de 5339,8 Th est conservé pour la masse-sur-charge retenue par la discrétisation de 5338 Th.

Un calcul en trois étapes similaires est effectué pour chacune des masses-sur-charges théoriques obtenues suite à la discrétisation. Ainsi, les figures **5b, 5e** et **5h** illustrent une convergence de la masse-sur-charge retenue par la discrétisation $M(j + 1, 0) = m(j + 1)$ d'une valeur de 5340 Th vers une valeur approximée de $M(j + 1, 3)$ de 5339,8 Th. De même, les figures **5c, 5f** et **5i** illustrent une convergence de la masse-sur-charge retenue par la discrétisation $M(j + 2,0) = m(j + 2)$ d'une valeur de 5342 Th vers une valeur approximée de $M(j + 2, 3)$ de 5339,8 Th.

A la suite de l'étape **244** d'approximation, le procédé se poursuit par à une étape **245** de suppression des masses-sur-charges approximées identiques.

Suite à l'approximation réalisée, une liste $\{m(j), \hat{M}(j)\}, j = 1, ..., J$ est obtenue. Du fait de la discrétisation initiale choisie de sorte à garantir un recouvrement des intervalles d'une masse-sur-charge par rapport à la suivante, plusieurs masses-sur-charges retenues $m(j)$ peuvent correspondre à une même masse-sur-charge approximée. Les approximations $\hat{M}(j)$ de ces masses-sur-charges sont dans ce cas égales ou presque égales en fonction de la précision retenue sur le calcul de la valeur. Le tableau 3 suivant illustre notamment la position des masses-sur-charges retenues et approximées dans l'intervalle 5338 à 5398 Th pour un exemple de mise en œuvre de l'invention avec un pas de discrétisation de 2 Th.

Tableau 3

| Position des masses-sur-charges m(j) | Position des masses-sur-charges approximée $\hat{M}(j)$ | Position des masses-sur-charges approximée $\hat{M}(j)$ conservée |
|---|---|---|
| 5338 | 5339.8 | |
| 5340 | 5339.8 | 5339.8 |
| 5342 | 5339.8 | |

(suite)

| Position des masses-sur-charges m(j) | Position des masses-sur-charges approximée $\hat{M}(j)$ | Position des masses-sur-charges approximée $\hat{M}(j)$ conservée |
|---|---|---|
| 5378 | 5381.2 | 5381.2 |
| 5380 | 5381.2 | |
| 5382 | 5381.2 | |
| 5384 | 5381.2 | |
| 5394 | 5397.4 | 5397.4 |
| 5396 | 5397.4 | |
| 5398 | 5397.4 | |

Une seule approximation est ainsi conservée pour chaque valeur.

Une nouvelle liste $R = \{R(k)\}$ ; $k = 1, ... , K$ ; $K \leq J$ des masses-sur-charges de référence du groupe est ainsi obtenue.

Selon une seconde alternative **250,** ces masses-sur-charges communes aux différents sous-groupes sont connues à priori. Cette connaissance peut par exemple être obtenue à partir de la liste de pics utilisés comme pics de référence pour la classification au niveau groupe. Ces pics étant connus pour représenter le groupe, ils ont une forte probabilité de pouvoir être utilisés comme masses-sur-charges de référence au sens de la présente invention. Ces masses-sur-charges peuvent également être connues par des analyses antérieures par spectrométrie de masse ou par d'autres méthodes analytiques et permettant de connaître la masse-sur-charge théorique d'un pic pour une molécule ou protéine caractéristique des différents sous-groupes et donc du groupe considéré.

Optionnellement et dans l'objectif d'améliorer la sélection de ces masses-sur-charges, une étape similaire à l'étape **242** de détection de la présence ou absence d'un ou de pics dans un intervalle de tolérance autour de chaque masse-sur-charge de référence connues à priori peut être effectuée. Cette étape **242** peut être suivie d'une étape similaire à l'étape **243** consistant à filtrer les masses-sur-charges en fonction de la fréquence de présence de pics par sous-groupes peut être effectuée.

La fréquence de présence d'un pic dans l'intervalle défini par la tolérance autours de chaque masse-sur-charge de référence connues à priori est calculée par sous-groupe et ramenée à un pourcentage.

Alternativement ou de façon complémentaire, cette étape **242** peut être suivie d'une étape similaire à l'étape **244** d'approximation de la position des masses-sur-charges de référence connues a priori peut être effectuée.

Une fois la liste des masses-sur-charges de référence obtenues à la suite de l'étape **240** ou **250,** le procédé se poursuit par l'ajustement des masses-sur-charges de l'ensemble des listes de pics dans l'étape **260** selon la figure **3a.**

Pour chaque spectre représenté par une liste de pics, l'objectif de l'étape **260** est d'ajuster les positions de tous les pics en apprenant un modèle de transformation à partir de la position des masses-sur-charges de référence. Les paramètres de ce modèle sont estimés de sorte que les pics observés sur le spectre coïncident au mieux avec la position approximée des masses-sur-charges de référence obtenues à l'issu de l'étape **240** ou avec la position théorique des masses-sur-charges de référence obtenues à l'issu de l'étape **250.**

Pour chaque spectre au format liste de pics :

- Soit $X = \{x(s)\}$ ; $s = 1, ... ,S$ la liste des masses-sur-charges des pics du spectre considéré
- Soit $R = \{R(k)\}$ ; $k = 1, ...., K$ la liste des masses-sur-charges de référence
- Soit $t_3$ le facteur de tolérance autour de la position de la masse-sur-charge $\{R(k)\}$, par exemple $t_3=0,0004$. La valeur du facteur de tolérance $t_2$ étant supérieure ou égale à $t_3$.

Pour chaque masse-sur-charge de référence $\{R(k)\}$, le procédé consiste à rechercher une masse-sur-charge parmi $\{x(s)\}$ , $s = 1, ... , S$ présente dans l'intervalle défini par la tolérance autour de la masse-sur-charge $\{R(k)\}$ , à savoir l'intervalle

$$[R(k) - R(k) * t_3 \,; R(k) + R(k) * t_3 \,]$$

Dans certains cas, où le décalage des masses-sur-charges du spectre est trop important ou par exemple lorsque les spectres ne comprennent que peu de pics, aucun pic n'est observé dans l'intervalle considéré.

Soit la séquence d'observations $\{R(l); x(l)\}$, $l \subseteq \{1, ... , K\}$ la liste des masses-sur-charges de référence $\{R(l)\}$ pour lesquelles un pic en position x(l) sur le spectre considéré a été observé. La transformation à appliquer aux masses-sur-charges du spectre est modélisée par le modèle $R = f(x)$ , le modèle $f$ pouvant être :

- un modèle de régression linéaire :

$$C = \beta_0 + \beta_1 x \ ;$$

$\beta_0$, $\beta_1$ étant les constantes du modèle
- un modèle de régression polynomial de degré 2 :

$$C = \beta_0 + \beta_1 x + \beta_2 x^2 \ ;$$

$\beta_0$, $\beta_1$, $\beta_2$ étant les constantes du modèle
- un modèle de régression non linéaire ou non-paramétriques, tel que des modèles de régressions locales de type Spline, Loess ou Lowess, des modèles de régression par noyau,...

Un modèle de régression linéaire est privilégié pour la mise en œuvre de l'invention afin de limiter l'erreur de prédiction lors de l'extrapolation du modèle en dehors du domaine de masses-sur-charges utilisé pour estimer les paramètres dudit modèle. La nécessité d'extrapoler apparaît par exemple lorsque les masses-sur-charges de référence sélectionnées ne couvrent qu'un sous ensemble du domaine des masses-sur-charges d'intérêt ou lorsque le décalage des masses-sur-charges du spectre considéré est trop important relativement à la tolérance $t_3$ considérée.

L'estimation des paramètres du modèle peut être réalisée par la méthode des moindres carrés ordinaires. Cependant, des valeurs aberrantes peuvent être observées sur certaines masses-sur-charges, dues par exemple à la spécificité de l'échantillon testé ou à un décalage initial des masses-sur-charges trop important sur une certaine zone de l'étendue de masse-sur-charge. Or, la méthode des moindres carrés est très sensible à la présence de valeurs aberrantes, même en faible nombre. Afin d'obtenir des estimations des paramètres non influencées par des points aberrants, il est préférable d'utiliser une méthode d'estimation dite robuste permettant de résoudre simultanément le problème de la détection des points aberrants et de l'estimation des paramètres du modèle. L'estimateur « biweight » de Tukey est ainsi privilégié pour la mise en œuvre de l'invention, préférentiellement résolu via l'utilisation de l'algorithme des moindres carrés pondérés itérés IRLS (« Iteratively Reweighted Least Squares » en langue anglaise). D'autres méthodes d'estimation robustes peuvent évidemment être envisagées, entre autre la méthode des moindres carrés médians (« LMS-Least Median of Squares » en langue anglaise), la méthode des moindres carrés tronqués (« LTS-Least Trimmed Squares » en langue anglaise) ainsi que toute méthode issue de la classe des M-estimateurs dont l'estimateur « biweight » de Tukey est un exemple particulier.

La position ajustée de tous les pics du spectre est ensuite inférée via le modèle appris précédemment sur les masses-sur-charges de référence. La correction des masses-sur-charges est ainsi extrapolée en dehors de l'intervalle des masses-sur-charges utilisées pour l'ajustement :

- Pour chaque masse-sur-charge $x(s)$, la masse-sur-charge ajustée est obtenue par $\hat{x}(s) = f(x(s))$
- On note $\hat{X}(s) = \{\hat{x}(s)\}$; $s = 1, ... S$ la liste des positions ajustées des pics du spectre

A la suite de l'étape d'ajustement **260,** une étape optionnelle **265** peut consister en l'optimisation de la liste des masses-sur-charges de référence basée sur la qualité de l'ajustement obtenu. L'objectif de cette étape est de s'assurer que la qualité de chaque masse-sur-charge de référence retenue est similaire entre les différents sous-groupes d'intérêt.

Pour chaque masse-sur-charge de référence R={R(k)} ; k=1,....,K ; K≤J et chaque sous-groupe : Le procédé comprend une étape de calcul de la fréquence de présence d'un pic pour chaque sous-groupe après ajustement des masses-sur-charges de chaque spectre dans l'intervalle défini par la tolérance $t_3$ autour de la masse-sur-charge R(k) . Cette fréquence constitue un premier indicateur.

A la suite de cette étape, le procédé comprend une étape de calcul de l'écart de la position des pics pour chaque sous-groupe après ajustement à la masse-sur-charge de référence, par exemple par le calcul de la médiane ou de la moyenne des résidus associés à la masse-sur-charge R(k). Cet écart constitue un second indicateur.

S'ensuit une étape de calcul de la dispersion de la position des pics pour chaque sous-groupe après ajustement par rapport à la masse-sur-charge de référence, par exemple par le calcul d'un écart-type, d'une étendue, ou encore d'un intervalle interquartile des résidus associés à la masse-sur-charge R(k). De façon générale, cette étape de calcul de dispersion peut être réalisée par toute méthode permettant de quantifier la dispersion des valeurs des positions des pics observés. Cette dispersion constitue un troisième indicateur.

A partir de ce calcul, l'étape **265** se poursuit par une étape de suppression de certaines masses-sur-charges de référence sur la base de la non-homogénéité d'au moins un des trois indicateurs entre les sous-groupes du groupe considéré.

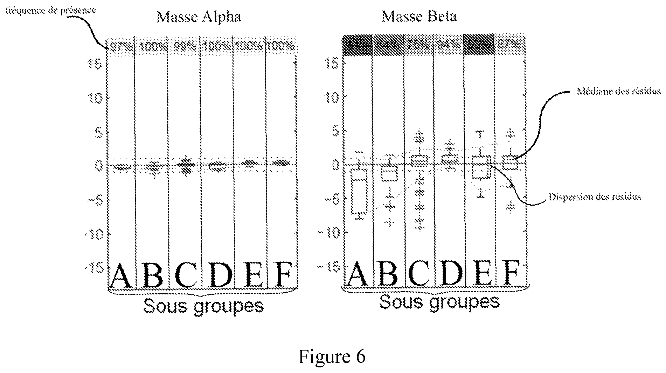

La Figure 6 illustre pour deux masses-sur-charges Alpha et Béta le calcul de :

- la fréquence de présence d'un pic pour chaque sous-groupe A à F
- la médiane des résidus pour chaque sous-groupe représentée par un trait horizontal à l'intérieur de chaque boîte à moustache
- l'intervalle interquartile des résidus pour chaque sous-groupe représenté par l'étendue de chaque boîte à moustache

Ainsi, ces trois indicateurs permettent par exemple de conserver la masse-sur-charge Alpha et d'écarter la masse-sur-charge Béta. En effet, la masse-sur-charge Alpha présente une fréquence de l'ordre de 100% entre les sous-groupes, une médiane des résidus proche de 0 pour chaque sous-groupe ainsi qu'une dispersion des résidus similaires entre chaque sous-groupe. Par contre, il est pertinent d'exclure la masse-sur-charge Béta car la fréquence de présence d'un pic est inférieure à 60% pour 2 sous-groupes, la médiane des résidus est décalée au-delà d'un seuil de 1 ou -1 pour le sous-groupe A, un seuil de médiane étant fixé à 1 ou -1 en pointillé. De plus, l'intervalle interquartile des résidus est nettement plus élevé pour les sous-groupes A et E. Le calcul de ces trois critères permet donc d'établir des seuils permettant d'écarter ou de conserver des masses-sur-charges de façon statistique.

L'étape **265** se termine alors par une étape de réajustement similaire à l'étape **260** mais réalisée uniquement à partir des masses-sur-charges retenues après l'étape de suppression de certaines masses-sur-charges de référence sur la base de la non-homogénéité d'au moins un des trois indicateurs entre les sous-groupes du groupe considéré.

De façon optionnelle, l'étape **260** ou l'étape **265** peuvent être suivies d'une étape **270** d'apprentissage et de construction d'un second modèle permettant l'ajustement des masses-sur-charges sur la gamme de masse-sur-charge d'intérêt pour la classification par sous-groupe.

L'étape **270** reprend l'étape **230** d'identification et de sélection de masses-sur-charges de référence communes aux différents sous-groupes et l'étape **260** d'apprentissage et de construction d'un modèle d'ajustement des masses-sur-charges afin de construire un second modèle d'ajustement à partir des listes de pics ayant déjà subis un premier ajustement, donc avec des décalages de masses-sur-charges supposés plus faibles.

La première étape d'ajustement, suite à l'étape **260,** peut en effet conduire à une extrapolation du recalage des masses-sur-charges sur certaines zones de l'étendue des masses-sur-charges d'intérêt suite à un décalage initial des masses-sur-charges important. Une deuxième étape d'apprentissage et de construction d'un second modèle permettant l'ajustement des masses-sur-charges via un modèle de régression polynomiale, par exemple d'ordre 2, peut être réalisée afin d'ajuster plus finement la position des pics sur une étendue plus large de masses-sur-charges. Pour cela les étapes **230,** et **260,** voire **265,** sont reproduites afin de sélectionner une liste de masses-sur-charges de référence communes aux différents sous-groupes et ajuster les masses-sur-charges de l'ensemble des listes de pics sur la gamme de masse-sur-charge d'intérêt pour la classification par sous-groupe.

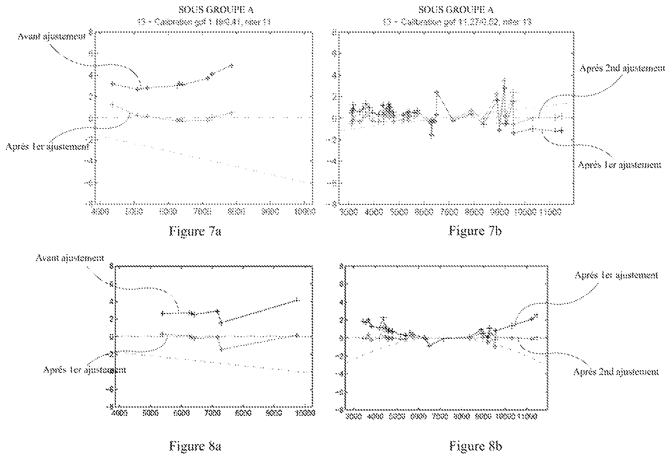

Les figures **7a** et **7b** illustrent l'intérêt de cette seconde étape d'ajustement.

La figure **7a** illustre le résultat d'un premier ajustement via un modèle de régression linéaire pour un spectre d'un sous-groupe donné A. La courbe noire représente l'écart entre la masse-sur-charge de référence et la position de la masse-sur-charge observée avant ajustement. La courbe grise représente quant à elle l'écart entre la masse-sur-charge de référence et la position de la masse-sur-charge après ajustement. Du fait d'un décalage initial élevé des masses-sur-charges, seules les masses-sur-charges de référence entre 4000 Th et 8000 Th ont été détectées. Le modèle de correction des masses-sur-charges est ensuite extrapolé en dehors de cet intervalle de masses-sur-charges sur l'ensemble des pics du spectre considéré. L'utilisation d'un modèle linéaire en première intention, permet de limiter l'erreur d'extrapolation.

La figure **7b,** illustre le résultat d'un deuxième ajustement du même spectre via un modèle de régression polynomial d'ordre 2. La courbe noire représente l'écart entre la masse-sur-charge de référence et la position de la masse-sur-charge observée après le premier ajustement, mais avant le second ajustement. La courbe grise représente quant à elle l'écart entre la masse-sur-charge de référence et la position de la masse-sur-charge après le deuxième ajustement. On constate que le modèle a été ajusté sur des masses-sur-charges détectées entre 3000 Th et 12000Th, permettant d'ajuster plus finement la position des pics sur une étendue de masses-sur-charges plus large. L'étape **270** peut éventuellement être répétée n fois afin de construire un n-ième modèle d'ajustement et ainsi améliorer l'ajustement des spectres.

L'étape suivante **280** consiste enfin en l'apprentissage et la construction d'une base de connaissance et à l'étape suivante **290** d'un algorithme de classification dédiés permettant la discrimination des sous-groupes à partir des listes de pics des spectres ayant subi l'ajustement ou les étapes d'ajustement des masses-sur-charges décrites ci-dessus.

La ou les étapes d'ajustement des masses-sur-charges ayant permis d'améliorer significativement la précision de la localisation des pics, l'algorithme de classification peut être :

- basé sur le calcul d'une distance tolérante, par exemple égale ou avantageusement plus faible que pour une classification au niveau groupe.

- basé sur une matrice de pics, obtenues par exemple par discrétisation des masses-sur-charges tel que décrit à l'étape **150.** Le pas utilisé pour la discrétisation des masses-sur-charges étant identique ou avantageusement plus fin que pour une classification au niveau groupe.

Tous les algorithmes de classification connus peuvent être utilisés, tels que la régression logistique, l'analyse discriminante, les arbres de classification, les méthodes de régression de type « LASSO » ou « elastic net », les algorithmes de type SVM (acronyme de l'expression anglo-saxonne « support vector machine »).

Le procédé selon l'invention permet donc d'obtenir un modèle d'ajustement des masses-sur-charges comprenant 1 à n listes de masses-sur-charges de référence et 1 à n modèles d'ajustement des masses-sur-charges ainsi qu'une base de connaissance et un algorithme de classification dédiés à la discrimination des sous-groupes du groupe considéré.

A partir de la base de connaissance et d'un algorithme de classification dédiés à la discrimination de groupes et de la base de connaissance et d'un algorithme de classification dédiés à la discrimination des sous-groupes d'au moins un groupe des groupes considérés, le procédé se poursuit par une étape de classification d'un microorganisme inconnu.

Cette étape de classification est par exemple mise en œuvre par un dispositif, comprenant :

- un spectromètre de masse apte à acquérir au moins un spectre de masse du microorganismes inconnu ;
- un système informatique apte à identifier le microorganismes inconnu en fonction du ou des spectres de masse acquis par le spectromètre, ledit système comprenant :

  - une mémoire informatique mémorisant au moins:

    o la base de connaissance et le modèle de classification par groupes de microorganismes ;
    o la base de connaissance et le modèle de classification par sous-groupes de microorganismes ;
    o le modèle d'ajustement pour la corrections de décalages de masse-sur-charge ;
    o des instructions informatiques pour la production d'une liste de pics à partir du spectre de masse acquis ;
    o des instructions informatiques pour la classification du microorganisme inconnu dans un groupe en fonction de la liste de pics produite selon ledit modèle de classification par groupes et ladite base de connaissance par groupes ;
    o des instructions informatiques pour l'ajustement de la liste de pics selon le modèle d'ajustement ;
    o des instructions informatiques pour la classification du microorganisme dans un sous-groupe en fonction de la liste de pics ajustée selon ledit modèle de classification par sous-groupes et ladite base de connaissance par sous-groupe ;

  - unité informatique à base de microprocesseur pour la mise en œuvre des instructions informatiques mémorisées dans la mémoire informatique de manière à classifier le microorganisme dans un groupe et un sous-groupe ;

une mémoire informatique pour mémoriser le résultat de la classification et/ou un écran d'affichage pour afficher le résultat de la classification.

Le procédé se poursuit donc, sur la figure **1,** par une étape **300** de classification par groupe. Comme décrit précédemment, cette étape se base sur la base de connaissance par groupe, et l'algorithme de classification par groupe associé, préexistants ou construits à partir d'un ensemble de spectres de microorganismes dont les groupes étaient préalablement identifiés.

L'étape **300** de classification par groupe débute, selon la figure **3c,** par une étape **310** d'acquisition d'au moins un spectre de masse dudit microorganisme inconnu. L'étape **310** débute par la préparation d'un échantillon du microorganisme inconnu à identifier, suivi de l'acquisition d'un ou plusieurs spectres de masse de l'échantillon préparé au moyen d'un spectromètre de masse par exemple un spectre de type MALDI-TOF. Cette étape est réalisée de façon similaire à l'étape **110.**

A la suite de l'étape d'acquisition, le procédé se poursuit par une étape **320** de détection des pics des spectres de façon similaire à l'étape **120** et d'étalonnage externe ou interne **330** de ces spectres, de façon similaire à l'étape **130.** Cette étape vise à obtenir un alignement des pics permettant la classification en groupe, dudit microorganisme. Comme présenté précédemment, la calibration externe consiste à ajuster l'axe m/z des spectres de masse d'un échantillon de référence, dont le contenu est connu et disposé à un point différent sur la plaque que l'échantillon, de manière à ce que les pics observés coïncident avec leur position théorique. La réalisation de cette étape est ainsi similaire à l'étape **130,** les pics du spectre du microorganisme inconnu étant réalignés en fonction de la transformation appliquée au spectre du calibrant.

A la suite de cette étape, le procédé comprend une étape **340** de classification de la ou des listes de pics obtenues. L'algorithme de classification par groupe, en relation avec la base de connaissance par groupe associé est pour cela mis en œuvre. Un ou plusieurs groupes (famille, germe, espèce,...) sont ainsi identifiés pour l'échantillon analysé.

Avantageusement et afin d'améliorer l'étape de classification par groupe, cette étape peut être précédée d'une étape de contrôle de la qualité des spectres de façon similaire à l'étape **140** ainsi qu'éventuellement d'une étape de discrétisation des masses-sur-charges, similaire à l'étape **150** et/ou d'une étape de traitement des intensités, similaire à l'étape **155.**

Alternativement, l'étape **340,** peut ne pas être réalisée dans le cas où le groupe du microorganisme analysé est connu mais le sous-groupe est inconnu. Dans ce cas, le procédé se poursuit directement à l'étape **350.**

Dans une étape suivante **350,** un résultat de l'étape de classification est obtenu, par exemple sous la forme d'un score de probabilité d'appartenance du microorganisme inconnu à un ou plusieurs groupes. Dans le cas où le groupe retenu ou au moins un des groupes retenus est représenté dans la base de connaissance par sous-groupe, le procédé selon l'invention se poursuit par une étape **400** de classification par sous-groupe.

Comme décrit précédemment, cette étape se base sur la base de connaissance par sous-groupe construite ainsi que sur l'algorithme de classification par sous-groupe associé, obtenus à partir d'un ensemble de spectres de microorganismes dont les groupes et sous-groupes étaient préalablement identifiés.

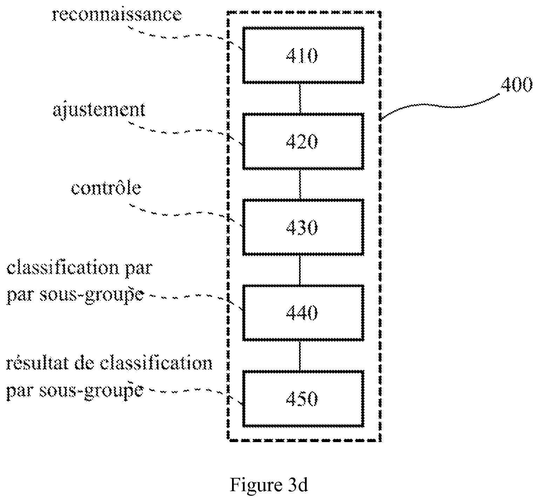

Selon la figure **3d,** l'étape **400** de classification par sous-groupe débute ainsi par une étape **410** de reconnaissance d'un résultat de classification de l'étape **350** d'un groupe pour lequel une base de connaissance par sous-groupe et un algorithme de classification par sous-groupe existe. Par exemple, un groupe taxonomique regroupant les espèces *Escherichia coli* et le genre *Shigella* peut être associé à une base de connaissance par sous-groupes taxonomique séparant les *Escherichia coli* non O157 (sous-groupe A), les *Escherichia coli* O157 (sous-groupe B), les espèces de *Shigella : Shigella dysenteriae* (sous-groupe C), *Shigella flexneri* (sous-groupe D), *Shigella boydii* (sous-groupe E), *Shigella sonnei* (sous-groupe F),etc...

L'étape suivante **420** consiste alors à ajuster les masses-sur-charges de la liste de pics obtenus à la suite de l'étape **330** à l'aide du modèle obtenu à la suite de l'étape **260,** et des masses-sur-charges de référence, caractéristiques du groupe, définies à l'étape **240** ou des masses-sur-charges de référeence, caractéristiques du groupe retenues à la suite de l'étape **250.** Dans le cas où un second modèle d'ajustement a été créé, la liste de pics est ensuite ajustée une seconde fois à l'aide du modèle d'ajustement obtenu à la suite de l'étape **270,** les masses-sur-charges caractéristiques utilisées étant alors celles du second modèle. De la même manière, dans le cas où un n-ième modèle d'ajustement a été créé, la liste de pics est ensuite ajustée une n-ième fois à l'aide du modèle d'ajustement obtenu à la suite de l'étape **270,** les masses-sur-charges caractéristiques utilisées étant alors celles du n-ième modèle.

Optionnellement, le procédé peut se poursuivre par une étape **430** de contrôle de la qualité de l'ajustement des masses-sur-charges. Pour cela, un nombre (ou un pourcentage) de masses-sur-charges de référence détectées sur le ou les spectres acquis peut être défini comme nécessairement supérieur à un seuil donné. Alternativement, ou complémentairement, une erreur quadratique moyenne RMSE (Root Mean Squared Error en langue anglaise) entre la position théorique de chaque masse-sur-charge de référence et la position après ajustement de ces masses-sur-charges sur le ou les spectres acquis peut être définie comme nécessairement inférieure à un seuil donné. Un calcul classique de l'erreur quadratique moyenne peut ainsi être obtenue par l'équation suivante :

$$\mathrm{RMSE} = \sqrt{\frac{1}{L}\sum_{l=1}^{L}(\hat{R}(l) - R(l))^2}$$

Où :

    ◦ {$R(l)$}, 1={1, ...L} la liste des L masses-sur-charges de référence pour lesquelles un pic a été observé sur le spectre considéré.

    ◦ f étant le modèle d'ajustement obtenu à la suite de l'étape **260,** éventuellement **270**

    ◦ $\hat{R}(l)$ étant la masse-sur-charge ajustée obtenue par $\hat{R}(l) = f(R(l))$,

A la suite de l'étape **420** ou **430,** le procédé se poursuit par une étape **440** de classification du spectre ajusté à partir de la base de connaissance par sous-groupe et de l'algorithme de classification permettant la discrimination des sous-groupes appris et définis préalablement.

Avantageusement et afin d'améliorer l'étape de classification par sous-groupe, cette étape peut être précédée d'une étape de discrétisation des masses-sur-charges, similaire à l'étape **150** et/ou d'une étape de traitement des intensités ; similaire à l'étape **155.**

Dans une étape suivante **450,** un résultat de l'étape de classification par sous-groupe est obtenu, par exemple sous la forme d'un score de probabilité d'appartenance du microorganisme inconnu à un ou plusieurs sous-groupes.

Le résultat de la classification pour groupe et sous-groupe, avantageusement avec leurs scores de classification, est stocké dans une mémoire informatique et/ou affiché sur un écran à l'attention de l'utilisateur.

**EXEMPLE DE CLASSIFICATION PAR SOUS-GROUPE D'UN GROUPE FORME PAR L'ESPECE *ESCHERICHIA COLI* ET LE GENRE *SHIGELLA*.**

Le procédé selon l'invention est appliqué à la classification de sérogroupes de l'espèce *Escherichia coli* et des espèces de *Shigella.* Le procédé vise ainsi à distinguer des sous-groupes en fonction de leur pathogénicité.

Le procédé utilise un spectromètre de masse MALDI-TOF VITEK® MS (bioMérieux , France) commercialisé par la demanderesse comprenant une base de connaissance par groupe VITEK® MS v2.0.0, également appelée base de donnée VITEK® MS v2.0.0 L'appareil VITEK® MS comprend également un algorithme de classification par groupe associé utilisant une classification multivariée associé à la base de connaissance par groupe. Un score d'appartenance à chacun des groupes étant obtenu à la suite de l'étape de classification par l'algorithme d'un spectre d'un microorganisme inconnu.

Le procédé selon l'invention permet ainsi de proposer une classification en deux étapes, par groupe puis par sous-groupe réalisable en routine sur un appareil de spectrométrie de masse. Tout d'abord, le groupe, ici un groupe taxonomique au niveau espèce serait identifié et dans le cas du groupe *Escherichia coli*/*Shigella* un second niveau de classification par sous-groupe est proposé pour différencier les 4 espèces de *Shigella* dudit groupe du sérogroupe O157 de l'espèce *Escherichia coli* et des sérogroupes non-O157 de l'espèce *Escherichia coli.*

Un premier lot A de 116 souches de microorganismes dont le groupe *Escherichia coli* et *Shigella* et les sous-groupes sont identifiés par des techniques classiques d'identification phénotypique et de sérotypage est créé. Ce lot sera utilisé pour la construction d'une base de connaissance et d'un modèle de classification par sous-groupe de référence.

Ce lot A contient :

- 60 souches *d'Escherichia coli* non O157 (référence **esh-col**) constituant le sous-groupe A
- 8 souches *d'Escherichia coli* de type 0157 (référence **esh-o157**) constituant le sous-groupe B
- 12 souches de *Shigella dysenteriae* (référence **shg-dys**) constituant le sous-groupe C
- 12 souches de *Shigella flexneri* (référence **shg-flx**) constituant le sous-groupe D
- 12 souches de *Shigella boydii* (référence **shg-boy**) constituant le sous-groupe E
- 12 souches de *Shigella sonnei* (référence **shg-son**) constituant le sous-groupe F

Ces 116 microorganismes ne sont pas distingués par l'appareil VITEK® MS actuel, l'algorithme de classification de l'appareil les classifiant ainsi dans le groupe « Escherichia coli / Shigella » de la base de connaissance associée.

Afin de procéder à l'acquisition des spectres de microorganismes du lot A par spectrométrie de masse, les échantillons contenant ces microorganismes sont préparés selon un protocole classique :

- Prélèvement d'une colonie après culture sur un milieu gélosé de croissance à l'aide d'une oese
- Remise en suspension de la colonie dans un tube Eppendorf de 2 mL contenant 300 μL d'eau déminéralisée
- Ajout de 0.9mL d'éthanol absolu et mélange (vortex)
- Centrifugation pendant 2 min à 10000 tours/min
- Elimination du surnageant à l'aide d'une pipette
- Ajout de 40 μL d'Acide Formique 70% et mélange (vortex)
- Ajout de 40 μL d'Acétonitrile et mélange (vortex)
- Centrifugation pendant 2 min à 10000 tours/min
- Dépôt de 1 μL de surnageant
- Séchage
- Ajout de 1 μL de matrice HCCA

Une quantité de chaque échantillon de chaque souche est déposée sur une plaque Maldi destiné à être utilisé avec l'appareil VITEK® MS. Les acquisitions sont réalisées en duplicat ou quadruplicat. L'acquisition est réalisée à l'aide du logiciel LaunchPad V2.8 et avec les paramètres suivants :

- Mode linéaire
- « Rastering : Regular circular »
- 100 profils/échantillon
- 5 tirs/profil
- Acquisition entre 2000 et 20000 Thomsons
- Paramètre auto-qualité activé

Suite à l'acquisition de ces spectres, l'appareil VITEK® MS procède au prétraitement et à la calibration externe à partir de l'acquisition de spectre d'une souche *d'Escherichia Coli* d'étalonnage (ATCC 8739) déposée sur l'emplacement réservé à la calibration du groupe d'acquisition. Une fois le spectre de la souche d'étalonnage acquis, la présence de 11 pics de références correspondant à des masses-sur-charges caractéristiques de *Escherichia Coli* est recherchée, avec une tolérance de 0.07 % autour de la position attendue des pics. Si au moins 8 pics sur les 11 se trouvent dans l'intervalle de position attendu, les pics du spectre de la souche d'étalonnage vont être réalignés en fonction de leur position de référence. La transformation obtenue est utilisée pour réaligner les spectres des échantillons acquis.

Un total de 388 spectres correspondant aux 116 souches du groupe du LOT A permettent ainsi la création d'une base de connaissance au niveau groupe et un algorithme de classification associé. Afin de confirmer que les microorganismes du LOT A ne sont pas distingués par l'appareil et appartiennent au même groupe pour la base de données VITEK® MS v2.0.0 et l'algorithme associé, il est procédé à une étape de classification par groupe. Les résultats de cette classification pour le lot A sont données dans le tableau 4 ci-dessous :

| Echantillons du lot A | Mauvais groupe identifié | Pas de groupe identifié | Groupe *Escherichia coli/Shigella* | Total |
|---|---|---|---|---|
| esh-col | | | 192 | 192 |
| esh-o157 | | | 31 | 31 |
| shg-boy | | | 39 | 39 |
| shg-dys | | | 32 | 32 |
| shg-flx | | 1 | 46 | 47 |
| shg-son | | | 47 | 47 |
| Total | 0 | 1 | 387 | 388 |

**Tableau 4**

99.7% des spectres du lot A sont correctement prédits comme appartenant au groupe *Escherichia coli/Shigella* de la base de données VITEK® MS v2.0.0. Un seul spectre obtenu à partir d'une souche de l'espèce *Shigella flexneri* n'est pas identifié, bien que de bonne qualité. Il est tout de même conservé pour la construction de la base de connaissance au niveau sous-groupe dans les étapes suivantes.

A partir de cette base de 388 spectres correspondant au lot A et au groupe *Escherichia coli/Shigella,* une base de connaissance au niveau sous-groupe ainsi qu'une méthode de classification associée sont créés.

Pour cela, l'ajustement des positions des masses-sur-charges des pics détectés est réalisée en deux étapes d'ajustement grâce à la construction successive de deux modèles d'ajustement. Dans une première étape d' ajustement, similaire à la réalisation des étapes **230, 240** et **260,** 10 masses-sur-charges caractéristiques du groupe, connues a priori, pour le groupe *Escherichia coli/Shigella* et situées entre 4000 et 10000 Th et correspondant aux masses-sur-charges du calibrant sont recherchées dans les 388 spectres. La tolérance autour de la position de ces masses-sur-charges sur chacun des spectres acquis est fixée à t = 0.0005%. A partir de la position observée de ces masses-sur-charges et de leur position théorique, un modèle de régression linéaire est calculé afin de les réaligner sur leur position théorique. La transformation obtenue est également appliquée à tous les pics de chacun des spectres acquis.

A la suite de cette première étape, une deuxième étape d'ajustement **270** est effectuée via un modèle de régression polynomiale d'ordre 2 ajusté sur une liste de masse-sur-charge de référence déterminée statistiquement selon le procédé décrit dans l'étape **240.** Pour cela, chacun des spectres ajustés à la suite de la première étape d'ajustement est discrétisé dans l'étendue de masses-sur-charges d'intérêt avec des pas de 1Th entre 3000 et 6000 Th, de 2 Th entre 6000 et 10000 Th et de 3 Th entre 10000 et 20000 Th. Chaque spectre est ainsi discrétisé en 8366 intervalles de masses-sur-charges. La présence ou l'absence de pics est recherché avec une tolérance de 0.0003% autour de chaque masse-sur-charge m(i) définies par la discrétisation selon le procédé décrit dans l'étape **242.** Les masses-sur-charges m(i) ainsi obtenues sont ensuite filtrées en fonction de la fréquence de présence de pics pour chacun des sous-groupes selon le procédé décrit dans l'étape **243.** 133 masses-sur-charges avec une fréquence minimale de présence pour chacun des sous-groupe de 60% sont ainsi retenues. Ceci permet de sélectionner des masses-sur-charges particulièrement caractéristiques du groupe.

La position de ces masses-sur-charges est ensuite approximée selon un modèle statistique de la position des masses-sur-charges retenues. Cette étape correspond à l'étape **244** décrite.

A partir des positions corrigées, les masses-sur-charges approximées identiques ou quasi-identiques, sont supprimées, afin de retenir une liste de 46 masses-sur-charges uniques, caractéristique du groupe. On considère que 2 masses-sur-charges après approximation sont identiques si l'écart observé entre les 2 masses-sur-charges est inférieur à 0.1Th. Cette étape correspond à l'étape **245** décrite.

**Tableau 5**

| Position des masses-sur-charges sélectionnées (discrétisation initiale) | Position des masses-sur-charges approximée (après ajustement) | Position des masses-sur-charges retenues |
|---|---|---|
| 5338 | 5339.8 | 5339.8 |
| 5340 | 5339.8 | |
| 5342 | 5339.8 | |
| 5378 | 5381.2 | 5381.2 |
| 5380 | 5381.2 | |
| 5382 | 5381.2 | |
| 5384 | 5381.2 | |
| 5394 | 5397.4 | 5397.4 |
| 5396 | 5397.4 | |
| 5398 | 5397.4 | |

Le tableau 5 précédent illustre sur l'intervalle de masses-sur-charges 5338 à 5398 Th la position des masses-sur-charges sélectionnées sur l'espace discrétisé des masses-sur-charges, la valeur approximée de ces mêmes masses-sur-charges et la liste finale des masses-sur-charges retenues après suppression des masses-sur-charges identiques.

Par la suite, une étape d'ajustement est ainsi réalisée de façon similaire à l'étape **270** à partir des positions des masses-sur-charges retenues. Une étape optionnelle permettant de contrôler et d'optimiser la liste des masses-sur-charges de référence basée sur la qualité d'ajustement obtenu permet de retenir une liste réduite de 37 masses-sur-charges finales de référence. Cette étape se base sur des critères tels que définis à l'étape **265.** Cinq masses-sur-charges sont éliminées car présentant pour au moins un des sous-groupes soit, un pourcentage de présence d'un pic après ajustement inférieur à 60%, soit une médiane des résidus supérieure à 1Th, soit un intervalle interquartile des résidus supérieur à 2Th. A partir de cette liste de masses-sur-charges de références réduites, le procédé se poursuit par un réajustement de l'ensemble des masses-sur-charges des listes de pics du groupe.

Selon la figure **8a,** le procédé comprend un premier ajustement similaire à l'étape **260** via un modèle de régression linéaire ajusté sur des masses-sur-charges de références détectées uniquement entre 5000 et 10000 Th du fait d'un décalage initial élevé des masses-sur-charges. La correction des masses-sur-charges est extrapolée en dehors de cet intervalle de masses-sur-charges. L'utilisation d'un modèle linéaire en première intention, permet de limiter l'erreur d'extrapolation sur la liste des masses-sur-charges du spectre considéré. Selon la figure **8b,** le procédé comprend un deuxième ajustement similaire à l'étape **270** via un modèle de régression polynomiale d'ordre 2 ajusté sur des masses-sur-charges détectées entre 3000 et 12000 Th, permettant d'ajuster plus finement la position des pics du spectre considéré sur une étendue de masse-sur-charge plus large.

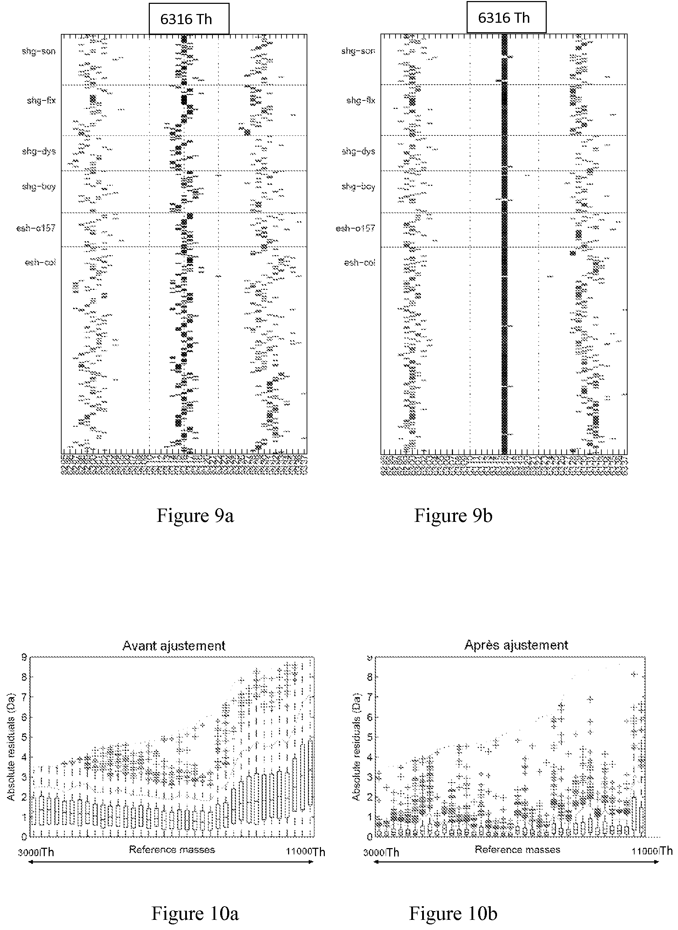

La figure 9a illustre pour une plage de masses-sur-charges la position des pics observée parmi tous les spectres du groupe et sous-groupe correspondant avant ajustement. La figure 9b illustre la position des mêmes pics après un second ajustement, démontrant la qualité de l'ajustement effectué ainsi que la pertinence de la masse-sur-charge sélectionnée comme masse-sur-charge de référence.

La précision revendiquée par le constructeur après calibration externe de l'appareil VITEK® MS est de 400 ppm, soit une précision en Thomson de l'ordre 1.2Th à 3000Th / 4.4Th à 11000 Th. La précision en Thomson observée après calibration externe, figure **10a,** est en médiane de l'ordre de la précision revendiquée sur le jeu de données considérés, à savoir de l'ordre de 1.2Th pour les masses-sur-charges vers 3000Th et de l'ordre de 3Th pour les masses-sur-charges vers 11000Th. Après le second ajustement des masses-sur-charges par le procédé selon l'invention, figure **10b,** la précision est de l'ordre de 0.12Th à 3000Th et de 0.44Th à 11000Th soit une précision de l'ordre de 40 ppm. Cette augmentation de la précision après ajustement par le procédé selon l'invention, démontre la pertinence des masses-sur-charges de référence sélectionnées et la qualité de l'ajustement réalisé.

Une base de connaissances et un algorithme de classification dédiés permettant la discrimination des sous-groupes du groupe *Escherichia coli/Shigella* à partir des listes de pics des spectres ayant subi l'ajustement décrits ci-dessus sont ensuite construits suivant le procédé décrit aux étapes **280** et **290.**

Pour cela il est construit une base de connaissances et un algorithme de classification dédié permettant la distinction des six sous-groupes suivants:

- Escherichia coli non 0157, sous-groupe A
- Escherichia coli O157, sous-groupe B
- Shigella dysenteriae, sous-groupe C
- Shigella flexneri , sous-groupe D
- Shigella boydii , sous-groupe E
- Shigella sonnei , sous-groupe F

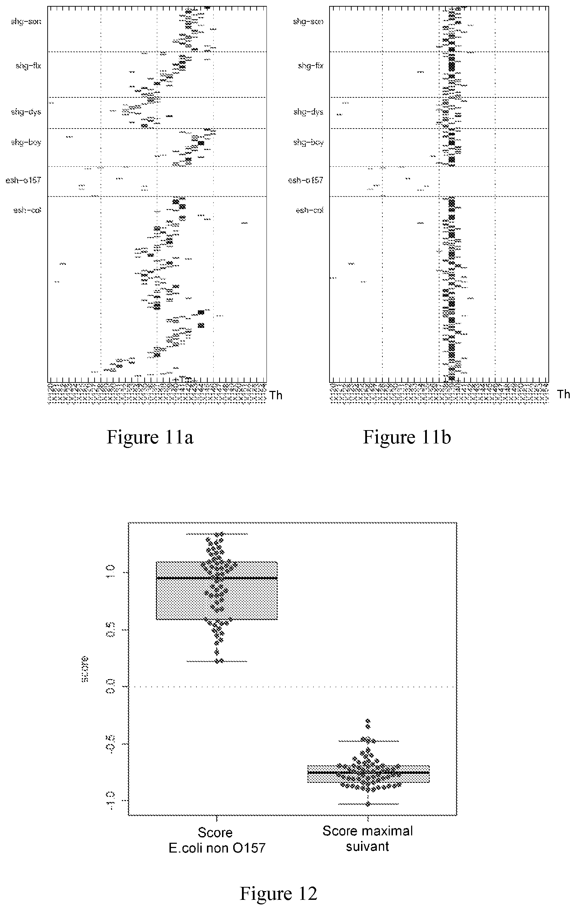

A titre d'exemple, la figure 11a illustre, pour une plage de masses-sur-charges contenant une masse permettant la discrimination du sous-groupe *Escherichia coli* O157 des autres sous-groupes, la position des pics observée parmi tous les spectres du groupe et sous-groupes correspondant avant ajustement. La figure 11b illustre la position des mêmes pics après un second ajustement, démontrant qu'il est alors possible d'utiliser la présence / absence du pic à 10139 Th avec une tolérance de +/- 2 Th pour détecter le sous-groupe *Escherichia Coli* de type O157 où ce pic est absent.

Afin de vérifier la capacité du modèle de classification et de la base de connaissance par sous-groupe associée à classifier des microorganismes par sous-groupe, un second lot B de 31 souches identifiées comme appartenant au groupe *Escherichia coli/Shigella* et dont les sous-groupes sont connus par des méthodes classiques d'analyse est également constitué.

Ce lot B, dit lot d'évaluation, contient 31 souches de *Shiga Toxine Escherichia Coli* (STEC) de 6 sérotypes O différents : O26, O45, O103, O111, O121 et O145.

Le protocole de préparation d'échantillon est identique à celui utilisé précédemment. Deux spectres sont acquis par souche afin d'obtenir une liste de 62 spectres repartis selon le tableau 6 suivant.

| Serotype O / Numéro ATCC | Nombre de spectres | Serotype O / Numéro ATCC | Nombre de spectres | Serotype O / Numéro ATCC | Nombre de spectres |
|---|---|---|---|---|---|
| O103 | 10 | O121 | 10 | O26 | 10 |
| BAA-2199 | 2 | BAA-2187 | 2 | BAA-2181 | 2 |
| BAA-2200 | 2 | BAA-2203 | 4 | BAA-2186 | 2 |
| BAA-2207 | 2 | BAA-2220 | 2 | BAA-2188 | 2 |
| BAA-2210 | 2 | BAA-2221 | 2 | BAA-2204 | 2 |
| BAA-2213 | 2 | | | BAA-2205 | 2 |
| O111 | 12 | O145 | 10 | O45 | 10 |
| BAA-179 | 2 | BAA-1652 | 2 | BAA-2185 | 2 |
| BAA-180 | 4 | BAA-2192 | 2 | BAA-2189 | 2 |
| BAA-184 | 2 | BAA-2211 | 2 | BAA-2191 | 2 |
| BAA-2180 | 2 | BAA-2222 | 2 | BAA-2198 | 2 |
| BAA-2201 | 2 | BAA-2223 | 2 | BAA-2202 | 2 |

## Tableau 6

Ces souches sont notamment identifiées dans la publication de l'American Type Culture Collection ATCC : "Big Six" Non-o157 Shiga Toxin-Producing Escherichia coli (STEC) Research Materials

Afin de confirmer que les microorganismes du lot B ne sont pas distingués par l'appareil et la base de connaissance de l'état de l'art et appartiennent ainsi au même groupe, il est procédé à une étape de classification par groupe selon l'étape 300. Les résultats de cette classification pour le lot B sont donnés dans le tableau 7 ci-dessous :

| Echantillons du lot B | Mauvais groupe identifié | Pas de groupe identifié | Groupe Escherishia coli/Shigella | Total |
|---|---|---|---|---|
| esh-col O103:H11 | | | 2 | 2 |
| esh-col O103:H2 | | | 4 | 4 |
| esh-col O103:H25 | | | 4 | 4 |
| esh-col O111:H8 | | | 12 | 12 |
| esh-col O121:H19 | | | 10 | 10 |
| esh-col O145:H25 | | | 2 | 2 |
| esh-col O145:H48 | | | 2 | 2 |
| esh-col O145:Nonmotile | | | 6 | 6 |
| esh-col O26:H11 | | | 10 | 10 |
| esh-col O45:H2 | | | 10 | 10 |
| Total | 0 | 0 | 62 | 62 |

**Tableau 7**

100% des spectres sont correctement prédits comme appartenant au groupe *Escherichia coli/Shigella* par l'algorithme de classification et la base de connaissance VITEK® MS v2.0.0

L'ensemble des spectres du lot B est conservé pour l'évaluation de la base de connaissance et l'algorithme de classification par sous-groupe selon l'étape **400.**

Le procédé selon l'invention est mis en œuvre à partir de la base de connaissance par sous-groupe préalablement créée ainsi que l'algorithme de classification associé. La classification attendue pour le Lot B est un résultat du type sous-groupe Escherichia coli non 0157.

Pour cela, les masses-sur-charges de la liste de pics obtenus durant l'étape de classification au niveau groupe sont ajustées à l'aide des premier et second modèle d'ajustement des masses-sur-charges définis préalablement.

Afin d'améliorer la performance de la classification, et de manière optionnelle, un contrôle qualité de l'ajustement des masses-sur-charges est effectué. Les critères de qualité définis pour assurer la qualité de l'ajustement des masses-sur-charges de chaque spectre sont les suivants:

- Pour le spectre considéré, au moins 28 masses-sur-charges doivent être détectées parmi les 37 masses-sur-charges de référence prédéfinies ainsi qu'une erreur quadratique moyenne RMSE (Root Mean Squared Error en langue anglaise) entre la position théorique de chaque masse-sur-charge de référence et la position après ajustement de ces masses-sur-charges sur le spectres acquis inférieure à 1.

5 spectres n'atteignent pas ces critères, 58 les atteignent.

Les 58 spectres retenus sont classifiés à partir de la base de connaissances et de l'algorithme de classification permettant la classification au niveau des sous-groupes définis préalablement. Comme illustré sur la **figure 12,** tous les spectres sont correctement identifiés au sous-groupe Escherichia coli non O157 avec des scores élevés. De plus, le deuxième meilleur score obtenu sur un autre sous-groupe est très nettement plus faible, ce qui assure la robustesse de la classification.

## Revendications

1. Procédé d'identification par spectrométrie de masse d'un sous-groupe de microorganisme inconnu parmi un ensemble de sous-groupes de référence, chaque sous-groupe appartenant à un groupe parmi un ensemble de groupes de référence, le procédé comportant :

    • Une première étape de construction d'une base de connaissance et d'un modèle de classification par groupe associé à partir d'un ensemble de spectres d'apprentissage de microorganismes identifiés comme appartenant auxdits groupes

    • Une seconde étape de construction d'une base de connaissance et d'un modèle de classification par sous-groupe associé à partir de l'acquisition d'au moins un ensemble de spectres d'apprentissage de microorganismes identifiés comme appartenant auxdits sous-groupes du groupe, la second étape comprenant pour chaque groupe de l'ensemble de groupes de référence :

  o La construction d'un modèle d'ajustement permettant la correction des décalages de masse-sur-charge des spectres d'apprentissage des sous-groupes du groupes à partir de masses-sur-charges de référence communes aux différents sous-groupes du groupe
  o L'ajustement des masses-sur-charges de l'ensemble des listes de pics des spectres d'apprentissage des sous-groupes du groupe
  o La construction d'un modèle de classification par sous-groupe et de la base de connaissance associé à partir des spectres d'apprentissage des sous-groupes ajustés

• Une troisième étape de classification à un sous-groupe d'un microorganisme inconnu comportant :

  o L'acquisition d'au moins un spectre du microorganisme inconnu
  o La classification dans un groupe dudit spectre selon ledit modèle de classification par groupe et ladite base de connaissance par groupe
  o L'ajustement des masses-sur-charges de l'ensemble de la liste de pics dudit spectre selon le modèle d'ajustement du groupe permettant la correction des décalages de masse-sur-charge du spectre du microorganisme inconnu
  o La classification de la liste de pics ajustée dans un sous-groupe dudit groupe par ledit modèle de classification par sous-groupe et la base de connaissance par sous-groupe

• Mémoriser le résultat de la classification et/ou afficher le résultat de la classification sur un écran d'affichage

2. Procédé d'identification selon la revendication 1, comprenant lors de l'étape de construction d'une base de connaissance et d'un modèle de classification par sous-groupe associé :

  • La construction d'un second modèle d'ajustement permettant la correction des décalages de masse-sur-charge des spectres acquis à partir de masses-sur-charges de référence communes aux différents sous-groupes
  • Une seconde étape d'ajustement des masses-sur-charges de l'ensemble des listes de pics des spectres d'apprentissage à partir du second modèle d'ajustement

3. Procédé d'identification selon la revendication 1 ou 2, comprenant une étape d'optimisation de la liste des masses-sur-charges de référence basée sur la qualité de l'ajustement obtenu suite à au moins une des étapes d'ajustement

4. Procédé d'identification selon les revendications 1 à 3, la construction d'un modèle d'ajustement utilisant une liste connue de masses-sur-charges de référence communes aux différents sous-groupes

5. Procédé d'identification selon la revendication 4, les masses-sur-charges de référence communes aux différents sous-groupes connues étant sélectionnées par une étape consistant à

  • Détecter la présence ou l'absence de pics autour des masses-sur-charges de référence selon un facteur de tolérance
  • Filtrer lesdites masses-sur-charges en fonction de la fréquence de présence de pics pour chacun des sous-groupes et/ou approximer la position des masses-sur-charges de référence retenues

6. Procédé d'identification selon les revendications 1 à 5, la construction d'un modèle d'ajustement utilisant une liste de masses-sur-charges de référence communes aux différents sous-groupes déduites selon des critères statistiques de fréquence de la présence des pics dans chacun des sous-groupes du groupe

7. Procédé d'identification selon la revendication 6, les masses-sur-charges de référence communes aux différents sous-groupes étant déduites par une étape consistant à

  • Discrétiser l'espace des masses-sur-charges de chacun des spectres de chaque sous-groupe
  • Détecter la présence ou l'absence de pics autour des masses-sur-charges définies par l'étape de discrétisation selon un facteur de tolérance
  • Filtrer lesdites masses-sur-charges en fonction de la fréquence de présence de pics pour chacun des sous-groupes
  • Approximer la position des masses-sur-charges retenues

8. Procédé d'identification selon la revendication 7, l'étape de discrétisation étant réalisée sur un intervalle de masses-sur-charges restreint par rapport à l'intervalle de masses-sur-charges obtenu suite à l'acquisition du spectre

9. Procédé d'identification selon l'une des revendications 5 à 8, l'étape d'approximation consistant à rechercher une position représentative de la répartition des positions des pics présents autour de chacune des masses-sur-charges retenues

10. Procédé d'identification selon l'une des revendications précédentes, l'étape de construction d'une base de connaissance et d'un modèle de classification par sous-groupe associé comprenant une étape de discrétisation des masses-sur-charges des spectres acquis

11. Procédé d'identification selon l'une des revendications précédentes, l'étape de construction d'une base de connaissance et d'un modèle de classification par sous-groupe associé comprenant une étape de traitement des intensités des spectres acquis

12. Procédé d'identification selon l'une des revendications précédentes, l'étape de construction d'une base de connaissance et d'un modèle de classification par sous-groupe associé comprenant une étape de contrôle de la qualité des spectres acquis

13. Procédé d'identification selon l'une des revendications précédentes, les paramètres du modèle ou des modèles d'ajustement étant obtenus par une méthode d'estimation dite robuste

14. Procédé d'identification selon l'une des revendications précédentes, les spectres acquis pour la première étape de construction d'une base de connaissance et d'un modèle de classification par groupe associé étant directement utilisés pour la seconde étape de construction d'une base de connaissance et d'un modèle de classification par sous-groupe associé, les groupes et sous-groupes des microorganisme d'apprentissage étant connus

15. Dispositif d'identification d'un sous-groupe de microorganisme par spectrométrie de masse, comprenant :

- un spectromètre de masse apte à produire des spectres de masse de microorganismes à identifier ;
- une unité de calcul apte à identifier un sous-groupe de microorganismes associé aux spectres de masse produits par le spectromètre en mettant en œuvre un procédé conforme à l'une quelconque des revendications précédentes.

16. Dispositif d'identification d'un sous-groupe de microorganisme par spectrométrie de masse, comprenant :

- un spectromètre de masse apte à acquérir au moins un spectre de masse d'un microorganismes à identifier ;
- un système informatique apte à identifier le microorganismes associé au au moins un spectre de masse acquis par le spectromètre, ledit système comprenant :

- une mémoire informatique mémorisant :

o une base de connaissance et un modèle de classification par groupes de microorganismes, associé à partir d'un ensemble de spectres d'apprentissage de microorganismes identifiés comme appartenant auxdits groupes ;
o une base de connaissance et un modèle de classification par sous-groupes de microorganismes, associé à partir de l'acquisition d'au moins un ensemble de spectres d'apprentissage de microorganismes identifiés comme appartenant auxdits sous-groupes du groupe;
o un modèle d'ajustement pour la corrections de décalages de masse-sur-charge des spectres acquis par le spectromètre de masse à partir de références communes aux différents sous-groupes de la base de connaissance et du modèle de classification par sous-groupes ;
o des instructions informatiques pour la production d'une liste de pics à partir du spectre de masse acquis du microorganisme inconnu ;
o des instructions informatiques pour la classification du microorganisme dans un groupe en fonction de la liste de pics produite selon ledit modèle de classification par groupes et ladite base de connaissance par groupes ;
o des instructions informatiques pour l'ajustement de la liste de pics selon le modèle d'ajustement ;
o des instructions informatiques pour la classification du microorganisme dans un sous-groupe en fonction de la liste de pics ajustée selon ledit modèle de classification par sous-groupes et ladite base de connaissance par sous-groupe ;

- unité informatique à base de microprocesseur pour la mise en œuvre des instructions informatiques mémorisées dans la mémoire informatique de manière à classifier le microorganisme dans un groupe et un sous-groupe ;
- une mémoire informatique pour mémoriser le résultat de la classification et/ou un écran d'affichage pour afficher le résultat de la classification.

**Patentansprüche**

1. Verfahren zur Identifizierung einer unbekannten Mikroorganismusuntergruppe aus einem Satz von Referenzuntergruppen mittels Massenspektrometrie, wobei jede Untergruppe einer Gruppe aus einem Satz von Referenzgruppen angehört, wobei das Verfahren umfasst:

   • einen ersten Schritt der Konstruktion einer Wissensdatenbank und eines assoziierten Modells zur Klassifikation nach Gruppe, basierend auf einem Satz von Trainingsspektren von Mikroorganismen, für die deren Angehörigkeit zu besagten Gruppen identifiziert wurde
   • einen zweiten Schritt der Konstruktion einer Wissensdatenbank und eines assoziierten Modells zur Klassifikation nach Untergruppe, basierend auf der Erfassung wenigstens eines Satzes von Trainingsspektren von Mikroorganismen, für die deren Angehörigkeit zu besagten Untergruppen der Gruppe identifiziert wurde, wobei der zweite Schritt für jede Gruppe des Satzes von Referenzgruppen umfasst:

      ○ die Konstruktion eines Anpassungsmodells zum Ermöglichen der Korrektur von Masse-zu-Ladungs-Abweichungen von Trainingsspektren von Untergruppen der Gruppe basierend auf Referenz-Masse-zu-Ladungs-Verhältnissen, die den verschiedenen Untergruppen der Gruppe gemeinsam sind
      ○ Anpassung von Masse-zu-Ladungs-Verhältnissen des Satzes an Peak-Listen von Trainingsspektren von Untergruppen der Gruppe
      ○ Konstruktion eines Modells zur Klassifikation nach Untergruppe und der assoziierten Wissensdatenbank basierend auf den angepassten Trainingsspektren von Untergruppen

   • einen dritten Schritt der Klassifikation eines unbekannten Mikroorganismus in eine Untergruppe, umfassend:

      ○ Erfassung wenigstens eines Spektrums des unbekannten Mikroorganismus
      ○ Klassifikation des Spektrums in eine Gruppe gemäß des Modells zur Klassifikation nach Gruppe und der Gruppen-Wissensdatenbank
      ○ Anpassung von Masse-zu-Ladungs-Verhältnissen des Satzes der Peak-Liste des Spektrums gemäß des Anpassungsmodells der Gruppe zum Ermöglichen der Korrektur von Masse-zu-Ladungs-Abweichungen des Spektrums des unbekannten Mikroorganismus
      ○ Klassifikation der angepassten Peak-Liste in eine Untergruppe der Gruppe mittels des Modells zur Klassifikation nach Untergruppe und der Untergruppen-Wissensdatenbank

   • Speichern des Ergebnisses der Klassifikation und/oder Anzeigen des Ergebnisses der Klassifikation auf einer Bildschirmanzeige.

2. Verfahren zur Identifizierung nach Anspruch 1, das beim Schritt der Konstruktion einer Wissensdatenbank und eines assoziierten Modells zur Klassifikation nach Untergruppe Folgendes umfasst:

   • die Konstruktion eines zweiten Anpassungsmodells zum Ermöglichen der Korrektur von Masse-zu-Ladungs-Abweichungen von erfassten Spektren basierend auf Referenz-Masse-zu-Ladungs-Verhältnissen, die den verschiedenen Untergruppen gemeinsam sind
   • einen zweiten Anpassungsschritt von Masse-zu-Ladungs-Verhältnissen des Satzes an Peak-Listen der Trainingsspektren basierend auf dem zweiten Anpassungsmodell.

3. Verfahren zur Identifizierung nach Anspruch 1 oder 2, umfassend einen Schritt der Optimierung der Liste der Referenz-Masse-zu-Ladungs-Verhältnisse auf Grundlage der Qualität der Anpassung, die nach wenigstens einem der Anpassungsschritte erhalten wird.

**4.** Verfahren zur Identifizierung nach den Ansprüchen 1 bis 3, wobei die Konstruktion eines Anpassungsmodells eine bekannte Liste von Referenz-Masse-zu-Ladungs-Verhältnissen, die den verschiedenen Untergruppen gemeinsam sind, verwendet.

**5.** Verfahren zur Identifizierung nach Anspruch 4, wobei die bekannten Referenz-Masse-zu-Ladungs-Verhältnisse, die den verschiedenen Untergruppen gemeinsam sind, ausgewählt werden durch einen Schritt, bestehend aus

• Detektieren des Vorkommens oder Fehlens von Peaks in der Nähe von Referenz-Masse-zu-Ladungs-Verhältnissen gemäß einem Toleranzfaktor
• Filtern der Masse-zu-Ladungs-Verhältnisse in Abhängigkeit von der Vorkommenshäufigkeit von Peaks für jede der Untergruppen und/oder Approximieren der Position der beibehaltenen Referenz-Masse-zu-Ladungs-Verhältnisse.

**6.** Verfahren zur Identifizierung nach den Ansprüchen 1 bis 5, wobei die Konstruktion eines Anpassungsmodells eine Liste von den verschiedenen Untergruppen gemeinsamen Referenz-Masse-zu-Ladungs-Verhältnissen verwendet, die gemäß statistischen Kriterien der Vorkommenshäufigkeit von Peaks in jeder der Untergruppen der Gruppe abgeleitet werden.

**7.** Verfahren zur Identifizierung nach Anspruch 6, wobei die Referenz-Masse-zu-Ladungs-Verhältnisse, die den verschiedenen Untergruppen gemeinsam sind, abgeleitet werden durch einen Schritt, bestehend aus

• Diskretisierung des Raumbereichs von Masse-zu-Ladungs-Verhältnissen von jedem der Spektren jeder Untergruppe
• Detektieren des Vorkommens oder Fehlens von Peaks in der Nähe von Masse-zu-Ladungs-Verhältnissen, welche durch den Diskretisierungsschritt definiert wurden, gemäß einem Toleranzfaktor
• Filtern besagter Masse-zu-Ladungs-Verhältnisse in Abhängigkeit von der Vorkommenshäufigkeit von Peaks für jede der Untergruppen
• Approximieren der Position der beibehaltenen Masse-zu-Ladungs-Verhältnisse.

**8.** Verfahren zur Identifizierung nach Anspruch 7, wobei der Diskretisierungsschritt an einem Intervall von Masse-zu-Ladungs-Verhältnissen durchgeführt wird, das gegenüber dem nach der Erfassung des Spektrums erhaltenen Intervall von Masse-zu-Ladungs-Verhältnissen eingegrenzt ist.

**9.** Verfahren zur Identifizierung nach einem der Ansprüche 5 bis 8, wobei der Approximationsschritt aus dem Ermitteln einer Position besteht, die für die Verteilung der Positionen von in der Nähe zu jedem der beibehaltenen Masse-zu-Ladungs-Verhältnisse vorkommenden Peaks repräsentativ ist.

**10.** Verfahren zur Identifizierung nach einem der vorhergehenden Ansprüche, wobei der Schritt der Konstruktion einer Wissensdatenbank und eines assoziierten Modells zur Klassifikation nach Untergruppe einen Schritt der Diskretisierung von Masse-zu-Ladungs-Verhältnissen der erfassten Spektren umfasst.

**11.** Verfahren zur Identifizierung nach einem der vorhergehenden Ansprüche, wobei der Schritt der Konstruktion einer Wissensdatenbank und eines assoziierten Modells zur Klassifikation nach Untergruppe einen Schritt der Behandlung von Intensitäten der erfassten Spektren umfasst.

**12.** Verfahren zur Identifizierung nach einem der vorhergehenden Ansprüche, wobei der Schritt der Konstruktion einer Wissensdatenbank und eines assoziierten Modells zur Klassifikation nach Untergruppe einen Schritt der Kontrolle der Qualität der erfassten Spektren umfasst.

**13.** Verfahren zur Identifizierung nach einem der vorhergehenden Ansprüche, wobei die Parameter des Anpassungsmodells oder der Anpassungsmodelle durch ein sogenanntes robustes Schätzverfahren erhalten werden.

**14.** Verfahren zur Identifizierung nach einem der vorhergehenden Ansprüche, wobei die Spektren, erfasst für den ersten Schritt der Konstruktion einer Wissensdatenbank und eines assoziierten Modells zur Klassifikation nach Gruppe, direkt für den zweiten Schritt der Konstruktion einer Wissensdatenbank und eines assoziierten Modells zur Klassifikation nach Untergruppe verwendet werden, wobei die Gruppen und Untergruppen der Trainings-Mikroorganismen bekannt sind.

15. Vorrichtung zur Identifizierung einer Mikroorganismusuntergruppe mittels Massenspektrometrie, umfassend:

  ■ ein Massenspektrometer, geeignet zum Erstellen von Massenspektren von zu identifizierenden Mikroorganismen;
  ■ eine Recheneinheit, geeignet zum Identifizieren einer Mikroorganismenuntergruppe, die mit den durch das Spektrometer erstellten Massenspektren assoziiert ist, mittels Durchführen eines Verfahrens gemäß einem der vorhergehenden Ansprüche.

16. Vorrichtung zur Identifizierung einer Mikroorganismusuntergruppe mittels Massenspektrometrie, umfassend:

  ■ ein Massenspektrometer, geeignet zum Erfassen von wenigstens einem Massenspektrum von einem zu identifizierenden Mikroorganismus;
  ■ ein Computersystem, geeignet zum Identifizieren des Mikroorganismus, der mit wenigstens einem durch das Spektrometer erstellten Massenspektrum assoziiert ist, wobei das System umfasst:

    - einen Computerspeicher zum Speichern von:

      ○ einer Wissensdatenbank und eines assoziierten Modells zur Klassifikation nach Gruppen von Mikroorganismen, basierend auf einem Satz von Trainingsspektren von Mikroorganismen, für die deren Angehörigkeit zu besagten Gruppen identifiziert wurde;
      ○ einer Wissensdatenbank und eines assoziierten Modells zur Klassifikation nach Untergruppen von Mikroorganismen, basierend auf der Erfassung von wenigstens einem Satz von Trainingsspektren von Mikroorganismen, für die deren Angehörigkeit zu besagten Untergruppen der Gruppe identifiziert wurde;
      ○ einem Anpassungsmodell für die Korrekturen von Masse-zu-Ladungs-Abweichungen der vom Massenspektrometer erfassten Spektren basierend auf den verschiedenen Untergruppen gemeinsamen Referenzen der Wissensdatenbank und des Modells zur Klassifikation nach Untergruppen;
      ○ Computerbefehlen für die Erstellung einer Peak-Liste basierend auf dem erfassten Massenspektrum des unbekannten Mikroorganismus;
      ○ Computerbefehlen für die Klassifikation des Mikroorganismus in eine Gruppe in Abhängigkeit von der erstellten Peak-Liste gemäß des besagten Modells zur Klassifikation nach Gruppen und der besagten Gruppen-Wissensdatenbank;
      ○ Computerbefehlen für die Anpassung der Peak-Liste gemäß des Anpassungsmodells;
      ○ Computerbefehlen für die Klassifikation des Mikroorganismus in eine Untergruppe in Abhängigkeit von der angepassten Peak-Liste gemäß des besagten Modells zur Klassifikation nach Untergruppen und der besagten Untergruppen-Wissensdatenbank;

    - eine Mikroprozessor-basierte Computereinheit zum Ausführen der im Computerspeicher gespeicherten Computerbefehle auf solche Weise, dass der Mikroorganismus in eine Gruppe und eine Untergruppe klassifiziert wird;
    - einen Computerspeicher zum Speichern des Ergebnisses der Klassifikation und/oder eine Bildschirmanzeige zum Anzeigen des Ergebnisses der Klassifikation.

**Claims**

1. A method for identifying by mass spectrometry an unknown microorganism subgroup among a set of reference subgroups, each subgroup belonging to one group among a set of reference groups, the method including:

  • A first step of constructing one knowledgebase and one classifying model per associated group on the basis of a set of learning spectra of microorganisms identified as belonging to said groups
  • A second step of constructing one knowledgebase and one classifying model per associated subgroup on the basis of the acquisition of at least one set of learning spectra of microorganisms identified as belonging to said subgroups of the group, the second step comprising, for each group of the set of reference groups:

    ○ Constructing an adjusting model allowing mass-to-charge offsets of the learning spectra of the subgroups of the group to be corrected on the basis of reference masses-to-charges that are common to the various subgroups of the group

◦ Adjusting the masses-to-charges of all of the lists of peaks of the learning spectra of the subgroups of the group
◦ Constructing one classifying model per subgroup and the associated knowledgebase on the basis of the adjusted learning spectra of the subgroups

• A third step of classifying to a subgroup an unknown microorganism including:

◦ Acquiring at least one spectrum of the unknown microorganism
◦ Classifying into a group said spectrum according to said per-group classifying model and said per-group knowledgebase
◦ Adjusting the masses-to-charges of all of the list of peaks of said spectrum according to the adjusting model of the group, allowing mass-to-charge offsets of the spectrum of the unknown microorganism to be corrected
◦ Classifying the adjusted list of peaks into a subgroup of said group with said per-subgroup classifying model and the per-subgroup knowledgebase

• Storing the classification result and/or displaying the classification results on a display screen.

2. The identifying method as claimed in claim 1, comprising in the step of constructing one knowledgebase and one classifying model per associated subgroup:

• Constructing a second adjusting model allowing mass-to-charge offsets of the acquired spectra to be corrected on the basis of reference masses-to-charges that are common to the various subgroups
• A second step of adjusting the masses-to-charges of all of the lists of peaks of the learning spectra on the basis of the second adjusting model

3. The identifying method as claimed in claim 1 or 2, comprising a step of optimizing the list of the reference masses-to-charges, which is based on the quality of the adjustment obtained following at least one of the adjusting steps

4. The identifying method as claimed in claims 1 to 3, the construction of an adjusting model using a known list of reference masses-to-charges that are common to the various subgroups

5. The identifying method as claimed in claim 4, the known reference masses-to-charges that are common to the various subgroups being selected with a step consisting in

• Detecting the presence or absence of peaks around reference masses-to-charges according to a tolerance factor
• Filtering said masses-to-charges depending on the frequency of presence of peaks for each of the subgroups and/or approximating the position of the retained reference masses-to-charges

6. The identifying method as claimed in claims 1 to 5, the construction of an adjusting model using a list of reference masses-to-charges that are common to the various subgroups and that are deduced according to statistical criteria of frequency of the presence of the peaks in each of the subgroups of the group

7. The identifying method as claimed in claim 6, the reference masses-to-charges that are common to the various subgroups being deduced with a step consisting in

• Discretizing the space of the masses-to-charges of each of the spectra of each subgroup
• Detecting the presence or absence of peaks around the masses-to-charges defined by the discretizing step according to a tolerance factor
• Filtering said masses-to-charges depending on the frequency of presence of peaks for each of the subgroups
• Approximating the position of the retained masses-to-charges

8. The identifying method as claimed in claim 7, the discretizing step being carried out over an interval of masses-to-charges that is restricted with respect to the interval of masses-to-charges that is obtained following the acquisition of the spectrum

9. The identifying method as claimed in one of claims 5 to 8, the approximating step consisting in seeking a position representative of the distribution of the positions of the peaks present around each of the retained masses-to-charges

10. The identifying method as claimed in one of the preceding claims, the step of constructing one knowledgebase and one classifying model per associated subgroup comprising a step of discretizing the masses-to-charges of the acquired spectra

11. The identifying method as claimed in one of the preceding claims, the step of constructing one knowledgebase and one classifying model per associated subgroup comprising a step of processing the intensities of the acquired spectra

12. The identifying method as claimed in one of the preceding claims, the step of constructing one knowledgebase and one classifying model per associated subgroup comprising a step of controlling the quality of the acquired spectra

13. The identifying method as claimed in one of the preceding claims, the parameters of the adjusting model or models being obtained with what is called a robust estimating method

14. The identifying method as claimed in one of the preceding claims, the spectra acquired for the first step of constructing one knowledgebase and one classifying model per associated group being directly used for the second step of constructing one knowledgebase and one classifying model per associated subgroup, the groups and subgroups of the learning microorganisms being known

15. A device for identifying a microorganism subgroup by mass spectrometry, comprising:

- a mass spectrometer able to produce mass spectra of microorganisms to be identified;
- a computing unit able to identify a subgroup of microorganisms associated with the mass spectra produced by the spectrometer by implementing a method as claimed in any one of the preceding claims.

16. A device for identifying a microorganism subgroup by mass spectrometry, comprising:

• a mass spectrometer able to acquire at least one mass spectrum of a microorganism to be identified;
• a computer system able to identify the microorganism associated with the at least one mass spectrum acquired by the spectrometer, said system comprising:

- a computer memory storing:

o one knowledgebase and one classifying model per group of microorganisms, associated based on a set of training spectra of microorganisms identified as belonging to said groups;
o one knowledgebase and one classifying model per subgroup of microorganisms, associate based on a set of training spectra of microorganisms identified as belonging to said subgroups of the group;
o an adjusting model for correcting mass-to-charge offsets of the spectra acquired by the mass spectrometer on the basis of references that are common to the various subgroups of the per-subgroup knowledgebase and classifying model;
o computer instructions for producing a list of peaks on the basis of the acquired mass spectrum of the unknown microorganism;
o computer instructions for classifying the microorganism into a group depending on the produced list of peaks according to said per-group classifying model and said per-group knowledgebase;
o computer instructions for adjusting the list of peaks according to the adjusting model;
o computer instructions for classifying the microorganism into a subgroup depending on the adjusted list of peaks according to said per-subgroup classifying model and said per-subgroup knowledgebase;

- a microprocessor-based computer unit for implementing computer instructions stored in the computer memory so as to classify the microorganism into a group and a subgroup;
- a computer memory for storing the result of the classification and/or a display screen for displaying the result of the classification.

Figure 1

acquisition → 110

détection des pics → 120

étalonnage → 130

contrôle → 140

discrétisation des masses → 150

traitement des intensités → 155

base de connaissance par groupe → 160

modèle de classification par groupe → 170

100

Figure 2

acquisition

210

détection des pics

220

construction d'un
modèle d'ajustement

230

200

masses-sur-charges de
référence déduites

240

250

masses-sur-charges
de référence connues

ajustement

260

contrôle / réajustement

265

construction d'un second
modèle d'ajustement

270

base de connaissance
par sous-groupe

280

modèle de classification
par sous-groupe

290

Figure 3a

discrétisation

présence / absence

filtrage

approximation

suppression

240

| 241 |
| 242 |
| 243 |
| 244 |
| 245 |

Figure 3b

acquisition

détection des pics

étalonnage

classification par
par groupe

résultat de classification
par groupe

300

| 310 |
| 320 |
| 330 |
| 340 |
| 350 |

Figure 3c

reconnaissance

410

400

ajustement

420

contrôle

430

classification par
par sous-groupe

440

résultat de classification
par sous-groupe

450

Figure 3d

Figure 4

Figure 5a

Figure 5b

Figure 5c

Figure 5d

Figure 5e

Figure 5f

Figure 5g

Figure 5h

Figure 5i

Figure 6

SOUS GROUPE A

Avant ajustement

Après 1er ajustement

Figure 7a

SOUS GROUPE A

Après 2nd ajustement

Après 1er ajustement

Figure 7b

Avant ajustement

Après 1er ajustement

Figure 8a

Après 1er ajustement

Après 2nd ajustement

Figure 8b

Figure 9a

Figure 9b

Figure 10a

Figure 10b

Figure 11a

Figure 11b

Figure 12

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **PETER KUHNERT et al.** Identification ofanimal Pasteurellaceae by MALDI-TOF mass spectrometry. *Journal of Mircobiological Methods*, 2012, vol. 89
- **ANNA RETTINGER et al.** Leptospira spp. strain identification by MALDI TOF MS is an equivalent tool to 16S rRNA gene sequencing and multi locus sequence typing (MLST). *BMC Microbiology*, 2012
- **CAROLE CASSAGNE et al.** Mould Routine Identification in the Clinical Laboratory by Matrix-Assisted Laser Desorption Ionization Time-Of- Flight Mass Spectrometry. *PLOS ONE*, 2011
- **JACKSON O. LAY**. Maldi-tof spectrometry of bacteria. *Mass Spectrometry Reviews*, 2001, vol. 20, 172-194